# EUROPEAN PATENT SPECIFICATION

(11) **EP 4 102 967 B1**
(45) Date of publication and mention of the grant of the patent: **25.11.2026**
(21) Application number: 21706689.3
(22) Date of filing: 12.02.2021
(51) Int. Cl.: A01N 1/143, A61B 46/10

(54) **APPARATUS FOR STORING AN ORGAN OR BODY TISSUE, AND CORRESPONDING METHOD**
VORRICHTUNG ZUM AUFBEWAHREN EINES ORGANS ODER KÖRPERGEWEBES UND ENTSPRECHENDES VERFAHREN
APPAREIL DE STOCKAGE D'UN ORGANE OU D'UN TISSU CORPOREL, ET PROCÉDÉ CORRESPONDANT

(30) Priority: 12.02.2020 GB 202001924; 12.02.2020 GB 202001925
(43) Date of publication of application: 21.12.2022
(73) Proprietor: University of Newcastle Upon Tyne, Newcastle Upon Tyne NE1 7RU (GB)
(72) Inventor: SCOTT III, William Earl, Newcastle upon Tyne NE1 7RU (GB); KAY, Stuart, Newcastle upon Tyne NE1 7RU (GB)
(74) Representative: Mathys & Squire
(86) International application number: PCT/GB2021/050351
(87) International publication number: WO 2021/161040

(56) References cited:
- WO-A1-2017/044861
- WO-A1-97/49370
- US-A1- 2013 263 867
- US-A1- 2015 342 177
- TRANSMEDICS: "Clinical User Guide: OCS(TM) Lung System", 1 January 2018 (2018-01-01), XP055801357, Retrieved from the Internet <URL:https://www.transmedics.com/wp-content/uploads/2019/03/100004071_REV5_OCS_Lung_System_-_Clinical_User_GuideSmall.pdf> [retrieved on 20210504]

## Description

### Technical Field

The present disclosure relates to apparatuses and methods for storing an organ or body tissue, such as for organ transplant surgery. This may include apparatuses and methods for preservation and/or transport of the organ or body tissue.

### Background

Gaseous oxygen perfusion or 'Persufflation' (PSF) is a technique for delivering oxygen via the native vasculature with the goal of maintaining the bio-energetic status of an organ or tissue. There are two main approaches to persufflation: anterograde persufflation (A-PSF), a physiological flow of gas entering through the artery and draining through the vein; and retrograde persufflation (R-PSF), where gas enters through the vein and exits through holes pricked into the surface of the organ or tissue with a needle.

US patent applications US2010/0330547 A1 and US 20120178150 A1 relate to perfusion devices based on electrochemical gas generation. Such devices are typically used in a clinical environment for the temporary storage of an organ or body tissue. US 2013/0263867 discloses a surgical drape. WO 97/49370 discloses a table for preparing transplantation tissue or organs, which includes a basin that is placed in an opening in the tabletop. A sterile cloth is placed on the tabletop, and will be entrained by the basin as the latter is lowered into the table opening. The cloth may include an opening whose defining edge seals tightly against an outwardly facing, peripherally extending outer wall-part of the basin.

### Summary of the invention

The invention provides an apparatus for storing an organ or body tissue as defined in independent claim 1, and a method of providing an organ or body tissue as defined in independent claim 15. Optional features are set out in the dependent claims.

In an aspect, there is thus provided an apparatus for storing an organ or body tissue, the apparatus comprising a container unit comprising a container body defining a storage region for storing an organ or body tissue, wherein the container body includes an opening to enable the organ or body tissue to be admitted to, and removed from, the storage region through the opening; and a surgical drape comprising: (i) a securing portion which is securable to the container unit, (ii) a peripheral outer portion which surrounds the securing portion, and (iii) a frangible portion separating the securing portion from the peripheral outer portion. The securing portion is securable to the container body to enable the drape to extend around a portion of the periphery of the opening with the peripheral outer portion of the drape arranged outward of the opening. The frangible portion extends around the securing portion to enable the peripheral outer portion to be separated from the securing portion by tearing the frangible portion.

Aspects may enable a sterile environment to be maintained in areas surrounding the organ. The drape may be used to maintain sterility of a surface beneath it (and/or to reduce the likelihood of contaminants associated with that surface coming into contact with the organ). Then, once the organ has been placed in the storage region of the container unit, the outer portion of the drape may be removed. This may inhibit the likelihood of the outer portion of the drape interfering with other parts of the storage apparatus. The outer portion of the drape may then be discarded, and the organ stored and/or transported when in the container unit (e.g. as inserted into a storage apparatus). The securing portion of the surgical drape may be secured to the container unit. A storage apparatus may be provided which comprises: (i) a fluid supply system comprising a store of persufflation fluid to be supplied to a said organ or body tissue, and (ii) a container unit receiving portion, wherein the container unit is removably insertable into the container unit receiving portion. A kit may be provided for the container unit and drape, and e.g. for the storage apparatus as well.

The container unit may be arranged so that the storage region defines a volume in which preservation fluid may be stored. A wall may extend around the periphery of the storage region (e.g. which defines the volume in which preservation fluid may be stored). This wall may also define the opening in the container unit. The securing portion of the drape may comprise a region of the drape which is adapted to be secured (e.g. attached) to a corresponding region of the container unit. The securing portion may comprise one or more attachment means to facilitate attachment to the container unit, e.g. the securing portion may comprise an adhesive. The securing portion may be sized to correspond to a corresponding attachment portion of the container unit. The frangible portion may extend circumferentially around the securing portion. Tearing the frangible portion may comprise tearing along the frangible portion. For example, it may comprise applying a force to the drape (e.g. by pulling the peripheral outer portion) which causes the frangible portion to tear away.

The drape may be circular (e.g. annular with an access opening in the middle), or it may take other shapes. Working radially outwards from the centre of the drape, the securing portion may be located radially inwards from the peripheral outer portion, e.g. with the frangible portion radially between these two. The frangible portion may have a weakness defined therein to facilitate easy tearing. For example, it may include a plurality of cuts where material has been removed, or where the material has been scored (e.g. by knife or laser). The drape may be arranged so that when installed on the container unit (with the securing portion secured to the container unit) the peripheral outer portion extends away from the opening. It is to be appreciated that the exact location of the securing portion, frangible portion and peripheral outer portion need not be considered limiting, as long as at least a portion of the peripheral outer portion is provided outwards from the opening to provide some protection. For example, a portion of the securing portion may also extend away from the opening, or alternatively both the frangible portion and the securing portion may be in contact with the container body (a portion of the peripheral outer portion may also be in contact with the container body).

The container unit may have an attachment portion to which the surgical drape is securable. The attachment comprise may comprise a wall, e.g. which extends around a region of the container unit to define the storage region and/or the opening. The attachment portion may surround at least a portion of the opening of the container unit. The securing portion of the drape may comprise an adhesive region for securing the drape to the container unit. The drape may include an access opening which corresponds to the opening of the container unit to enable surgical drape to be secured to the container unit with the access opening in alignment with the opening of the container unit. The access opening may comprise a hole of the same size as, or larger than, the size of the opening hole. The access opening may be arranged to enable the drape to be placed over the container unit with the opening being exposed through the access opening (e.g. to enable a user to place the organ through both openings into the storage region of the container unit). The adhesive region may surround at least a portion of the access opening. The drape may be arranged to surround the opening, such as by circumscribing, but not necessarily completely circumscribing, the opening. At least a portion of the peripheral outer portion may be arranged outward of the opening (e.g. radially outward) to provide protection for a surface underneath that portion of the drape. For example, the peripheral outer portion may be arranged about the opening.

The surgical drape may comprise a first surgical drape. The apparatus may further comprise a second surgical drape (e.g. the apparatus may include two separate drapes). The second surgical drape may comprise a second securing portion which is securable to the container body to enable the second drape to extend around a portion of the periphery of the opening. Both drapes may be attached to the container unit, e.g. to different parts of the container unit. The container unit may comprise a second attachment portion to which the second surgical drape is securable. The second attachment portion may be different to the first portion. The second attachment portion may comprise an inner surface of a wall of the container body. For example, a wall may extend around the storage region and/or the opening, and the second attachment portion may comprise a portion of that wall, such as on the inside (e.g. radially) of the wall.

The container unit may further comprise an inner lid member which is configured to be secured to the container body so as to enclose the organ or body tissue within the storage region between the container body and the inner lid member. The second attachment portion may be an inner surface of a wall of a container unit which is external the storage region enclosed by the lid member and the container body. The apparatus may be arranged to enable the second drape to be secured to the container body so that a portion of the second drape overlies the inner lid member. The container unit may comprise an outer lid member which is configured to be secured to the container body so as to enclose the inner lid member between the container body and the outer lid member.

It will be appreciated in the context of the present disclosure that apparatuses for storing an organ or body tissue disclosed herein may be provided assembled or unassembled. For example, different components of the apparatus may be attached to other components within the apparatus, or they may be arranged to be attached, but are not provided attached.

In another aspect, there is provided a method of providing an organ or body tissue in the apparatuses for storing an organ or body tissue disclosed herein. The method comprises: placing the organ or body tissue through the opening in the container unit and into the storage region of the container unit; and tearing along the frangible portion of the drape to remove the peripheral outer portion of the drape after the organ or body tissue has been placed into the storage region of the container unit. The method may comprise attaching an inner lid member to the storage region of the container unit. It may comprise attaching a second drape so that a portion of the second drape overlies the inner lid member. The method may comprise removing the organ or body tissue from the storage region after tearing the frangible portion.

### Figures

Some examples of the present disclosure will now be described, by way of example only, with reference to the figures, in which:
Figure 1 shows a portable apparatus for storing an organ or body tissue in a closed configuration;
Figure 2 is a sectional view of the portable apparatus shown in Figure 1;
Figure 3 is a sectional view of a portion of the portable apparatus shown in Figures 1 and 2 in an open configuration;
Figure 4 is a schematic representation of a layout of a portable apparatus;
Figure 5 is a plan view of a portion of a container unit;
Figure 6 is sectional view along A-A of Figure 5;
Figure 7 is a sectional view along B-B of Figure 5;
Figure 8A shows a plan view of a fluid processing device which also illustrates the location of some hidden features;
Figure 8B is a sectional view along B-B of Figure 8A;
Figure 8C is an end view of the fluid processing device shown in Figure 8A;
Figure 8D is an end view of the fluid processing device shown in Figure 8A;
Figure 8E is a side view of the fluid processing device shown in Figure 8A;
Figure 8F is a sectional view of the fluid processing device shown in Figure 8A from the underside;
Figure 9 shows an alternative example of a fluid processing device;
Figure 10 shows a base unit in which the base unit is in an open configuration with a lid removed;
Figure 11 shows a surgical drape;
Figure 12A is a schematic representation showing a partial sectional view of a portion of a base unit and a container unit in a first configuration;
Figure 12B corresponds to Figure 12A and shows the base unit and the container unit in a second configuration;
Figure 12C corresponds to Figure 12A and shows the base unit and the container unit in a third configuration;
Figure 12D corresponds to Figure 12A and shows the base unit and the container unit in a fourth configuration;
Figure 12E corresponds to Figure 12A and shows the base unit and the container unit in a fifth configuration;
Figure 12F corresponds to Figure 12A and shows the base unit and the container unit in a sixth configuration;
Figure 13A corresponds to Figure 12A and shows the base unit and the container unit in an seventh configuration;
Figure 13B corresponds to Figure 12A and shows the base unit and the container unit in a eighth configuration;
Figures 14A shows a cooperation between a base unit and a first container unit;
Figure 14B shows cooperation between a base unit and a second container unit;

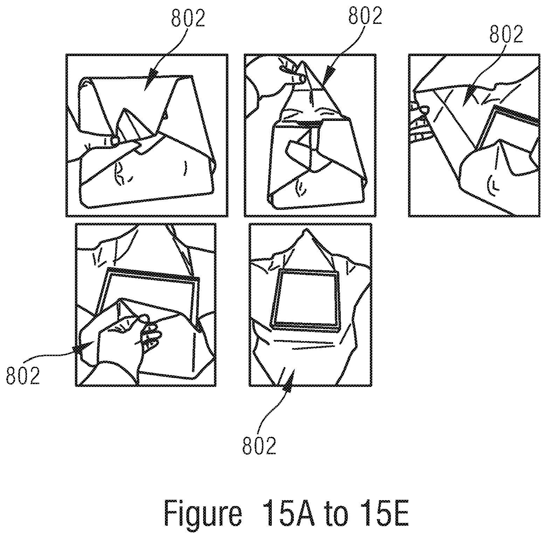

Figures 15A to 15E (numbered from left to right and top to bottom) show stages of a surgical drape being unfolded;
Figure 16 is a schematic representation of a portion of the apparatus shown in Figures 1 and 2 during use;
Figure 17 shows a user interface;
Figure 18 shows components of a system for persufflating an organ or body tissue;
Figure 19 illustrates a method of persufflating an organ or body tissue;
Figure 20 is a schematic illustration of a portion of an altemative example of a container unit;
Figure 21 is a projection view of a portion of a flow passageway shown in Figure 20;
Figure 22 is a schematic representation of a portion of an example of a container unit;
Figure 23A is a schematic representation of a layout of certain components of a container unit;
Figure 23B is a schematic representation of a layout of certain components of a container unit;
Figure 23C is a schematic representation of a layout of certain components of a container unit;
Figure 24 is a schematic representation of a portion of a container unit; and

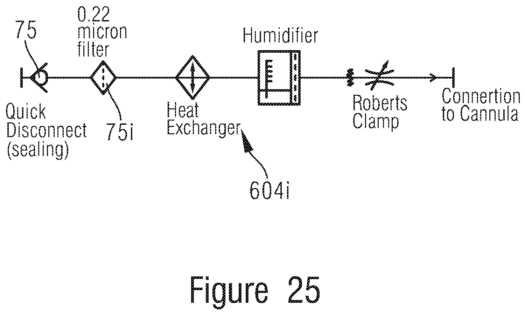

Figure 25 is a schematic representation of a portion of a system comprising certain components of a container unit.

In the drawings like reference numerals are used to indicate like elements.

### Specific Description

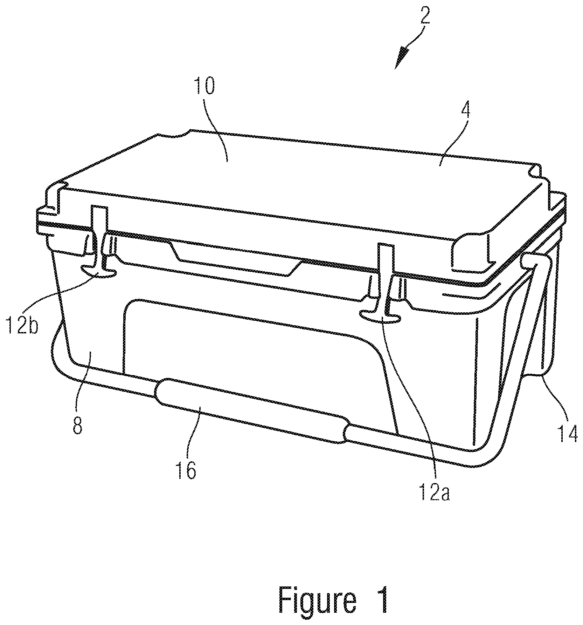

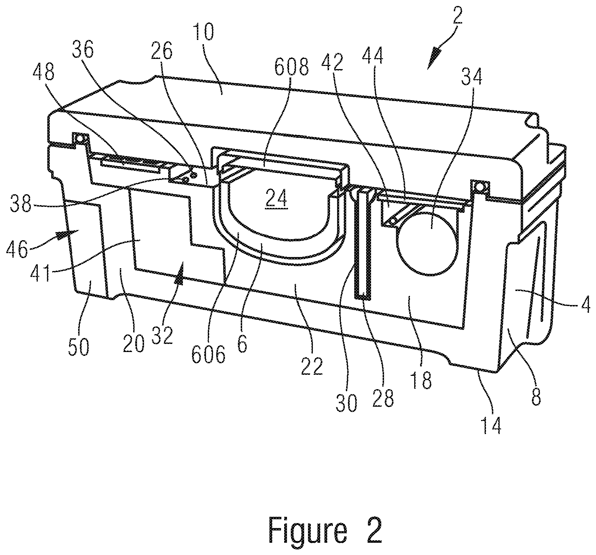

Figures 1 and 2 show a portable storage apparatus 2 for storing an organ or body tissue of a human or animal. The apparatus 2 comprises a base unit 4 and a container unit 6 (visible in Figure 2). The base unit 4 (which may also be referred to as a retained unit) comprises a lower portion 8 and a detachable base unit lid member 10 having clasps 12a, 12b which secure the lid to the lower portion. The base unit 4 is generally oblong and has a generally flat lower surface 14 on which the base unit 4 is supported. The base unit 4 also has a carrying handle 16 which is connected at opposite ends of the lower portion 8.

The base unit 4 comprises an inner portion 18 and an outer portion 20. The inner portion 18 comprises a docking portion 22 which is configured to receive the container unit 6. The docking portion 22 defines a storage region in the form of a storage cavity 24 having an opening 26 at the top of the storage cavity 24 through which the container unit 6 can be inserted into the storage cavity 24.

Cooling elements 28 in the form of ice-packs are arranged adjacent the docking portion 22 in order to passively cool the container unit 6 once it is received within the storage cavity 24. The cooling elements 28 are housed within respective vertical slots 30 provided in the inner portion 18 such that the cooling elements 28 can be inserted and removed from the slots 30, as required.

The base unit 4 further comprises a fluid supply system 32. The fluid supply system 32 comprises a fluid source 34 connected to a first outlet port 36, a second outlet port 38, a third outlet port 40 and at least one regulator and associated conduits (indicated generally by reference 41 and described later in further detail). The fluid supply system 32 may optionally include a fluid processor such as a humidifier and/or a cooling/heating element. The cooling elements 28 (and heating elements if provided) may be arranged to cool the persufflating gas as it passes through the fluid supply system 32 prior to reaching the outlet ports 36, 38, 40. The fluid source 34 comprises a gas canister which is located within a fluid source storage cavity 42 defined by the inner portion 18. The fluid source storage cavity 42 is provided with a removable transparent cover 44.

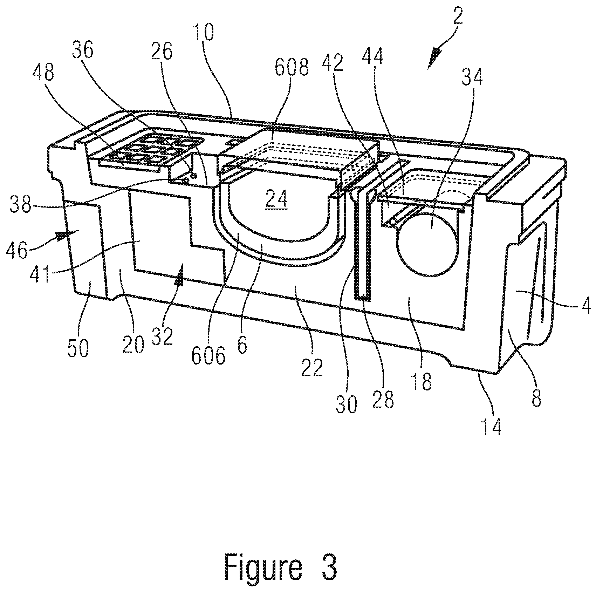

Figure 3 shows the base unit 4 with the lid member 10 removed so that the top of the lower portion 8 is visible. The base unit 4 further comprises a control system 46 which comprise a user interface 48 in the form of key pad, a controller and power source (indicated generally by reference 50). The control system is configured to monitor and control portable apparatus 2. The heat generating components of the control system, such as the power source, are located externally of the outer portion 20.

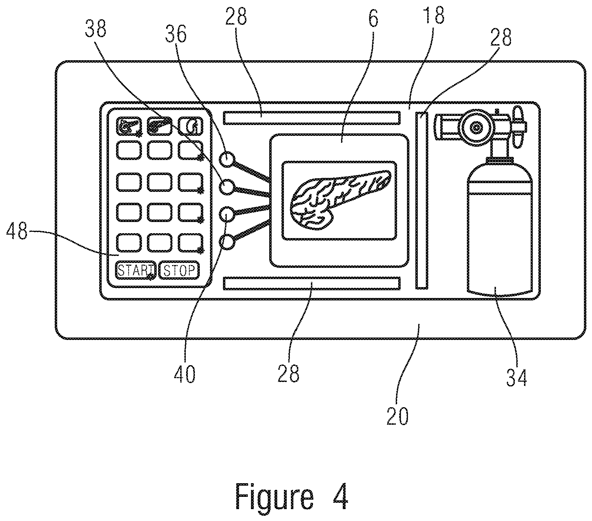

Figure 4 shows an example layout of the base unit 4 in which like reference figures are used to refer to like parts.

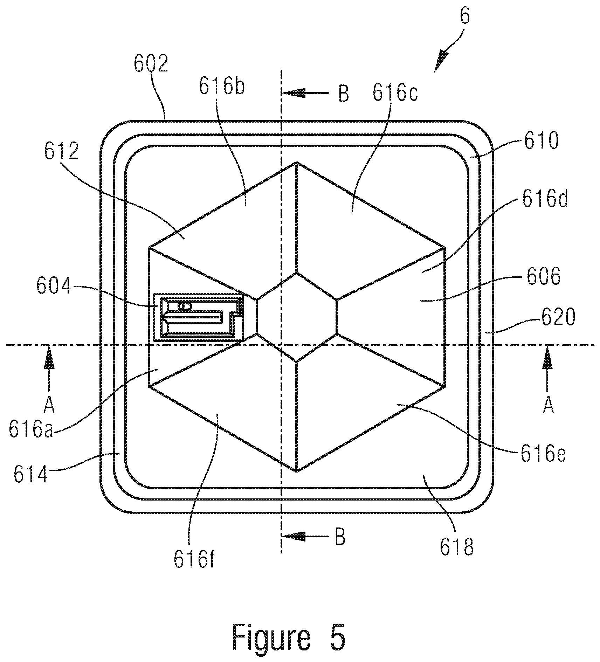

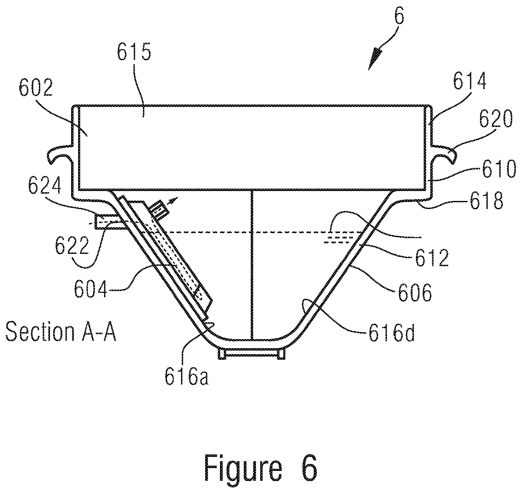

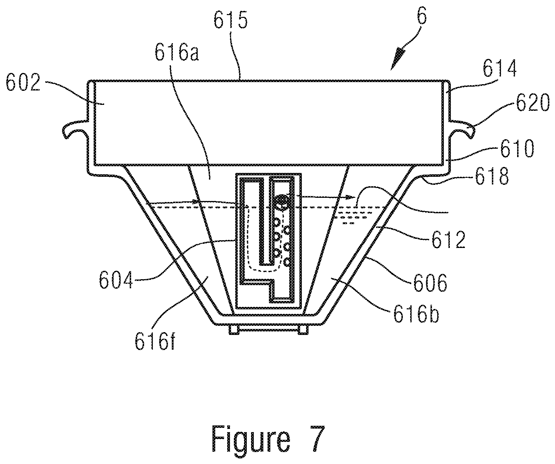

Figures 5, 6 and 7 show the container unit 6 in more detail. The container unit 6 (which may also be referred to as a disposable unit or a consumable unit or a primary organ container) comprises a container body 602 and a fluid processing device 604. The container body 602 comprises a storing portion 606 and a container unit lid member 608 (not shown in Figures 5, 6 or 7, but shown as part of a general arrangement in Figures 2 and 3).

The storing portion 606 has an outer wall 610 which forms a frusto-conical lower portion 612 and an upper portion 614 and defines an opening 615 through which an organ can be inserted or removed from the storing portion 606. It will be appreciated that other shapes for the storing unit could be used such as hemispherical or square or ellipsoidal or the like.

The lower portion 612 forms a bowl which defines a cavity in which the organ or body tissue is stored. The lower portion 612 has a hexagonal cross-section (when viewed from the top) and defines six substantially flat inner surfaces 616a, 616b, 616c, 616d, 616e, 616f.

The upper portion 614 has a substantially rectangular cross-section (when viewed from the top). A connection portion 611 of the outer wall 610 forming the upper portion 614 extends horizontally to form a ledge 618. The upper portion 614 also comprises a retaining lip 620 to which the container unit lid member 608 is secured.

A container inlet port 622 is provided through the outer wall of the lower portion 612. The inlet port 622 is located immediately below the ledge 618 and comprises a connector 624 which extends outwardly from the side of the portion of the outer wall 610 forming the lower portion 612. It will be appreciated that the connector can extend further or less distance in length than that shown and can terminate in a connection element such as a male or female part of a quick disconnect connector.

The storing portion 606 is formed from a single piece of material, such as a polypropylene or other relatively inexpensive plastic. Similarly, the container unit lid member 608 is also formed from a single piece of material, such as a polypropylene or other relatively inexpensive plastic. In the present example the storing portion 606 and the container unit lid member 608 are each formed by a moulding process.

The fluid processing device 604 is fixed to one of the inner surfaces 616a of the lower portion 612, for example by an adhesive, ultrasonic weld or other suitable means.

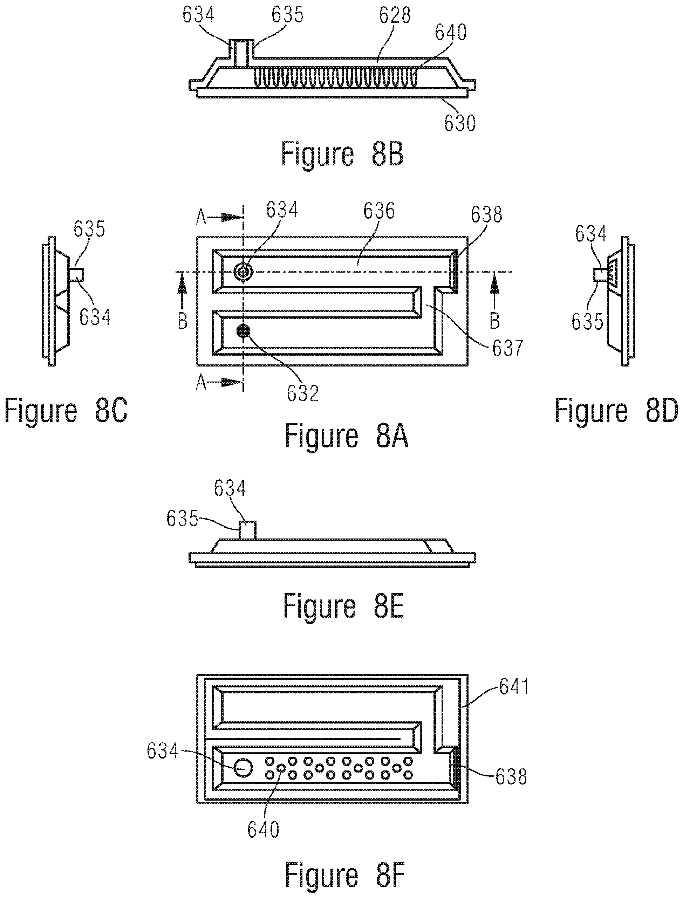

The fluid processing device 604 is shown in isolation in Figures 8A to 8F.

The fluid processing device 604 comprises an upper wall 628 and a bead 630, as shown in Figure 8B. The bead 630 is secured via a fluid tight seal to a portion of the inner surface of the wall of the lower portion 612 of the wall of the storage portion. Alternatively the fluid processing device can have a lower base plate. A first inlet port 632 is provided though the lower wall and an outlet port 634 is provided through the upper wall 628. The outlet port 634 comprises a connector 635 for connecting to a delivery device such as a delivery tube and cannula arrangement. The first inlet port 632 and the outlet port 634 are provided in fluid communication with each other by an internal passageway 636 defined between the upper wall 628 and the lower wall 630.

The internal passageway 636 has a serpentine arrangement, meaning that the internal passageway 636 comprises at least one bend through which the internal passageway 636 turns through at least 90 degrees. In the example shown, the passageway 636 turns through a bend 637 of 180 degrees. The fluid processing device 604 is elongate and the first inlet port 632 and the outlet port 634 are provided at the same end of the of the fluid processing device 604 and the internal passageway 636 extends from the first inlet port 632 along the fluid processing device 604 then turns through 180 degrees and extends back along the fluid processing device 604 to the outlet port 634. It will be appreciated that as an alternative the internal passageway may have a linear arrangement.

A second inlet port 638 is provided at the end of the fluid processing device 604 which is opposite the end at which the first inlet port 632 and the outlet port 634 are provided. The second inlet port 638 is an elongate slot (termed a "letterbox") which is arranged to allow fluid to enter the internal passageway 636 in the region of the turn 637 of the internal passageway 636. Other shapes of port can be utilised.

Baffles 640 are provided in the region of the internal passageway 636 which extends from the bend 637 to the outlet port 634. Each baffle 640 comprises a cylindrical pin (but may be a conical frustra) which extends from the upper wall 638 towards the lower wall 630. In the example shown, the baffles 640 are formed integrally with the upper wall 638 and each baffle 640 extends across substantially all of the internal passageway 636 in a direction which is perpendicular to the upper wall 638. It will be appreciated that the baffles 640 may be formed integrally with the upper wall 638 and/or the lower wall 630 and may extend partially across the internal passageway 636. The internal passageway 636 (regardless of whether it is straight or bent once or u-shaped or serpentine) thus includes one or more elements that break up any bubbles provided by the incoming gas. The bubble break up elements can be pegs or beads or plates or hemispherical or tapered projections or other such elements that operate to create a longer pathway in the internal passageway and/or provide shattering surfaces and/or define interspatial gaps that preclude transfer of large bubbles.

As the bowl-like storage region in the container unit fills with a preservation liquid the liquid automatically fills the space defined by the internal passageway. The passageway and fluid processing device in general thus forms an integral part of the disposable container unit.

The upper wall 628 and a lower wall 630 may be joined at their peripheries by a sealing bead 641, such as a bead formed by ultrasonic welding of the upper wall 628 and a lower wall 630 together as shown in Figure 8F.

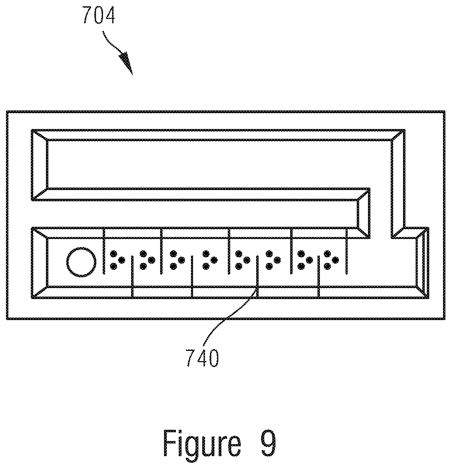

Figure 9 shows an alternative example of a fluid processing device 704 in which baffles 740 are arranged to define a zig-zag flow path along a flow passageway.

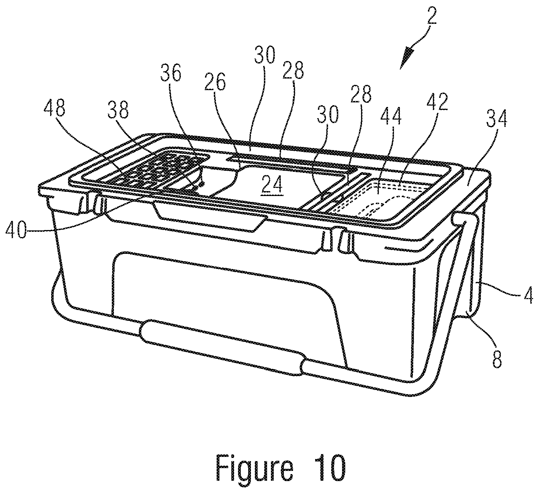

Prior to use, the base unit lid member 10 is removed from the lower portion 8 of the base unit 4, as shown in Figure 10. The container unit 6 is then inserted into the storage cavity 24, as shown in Figure 3 so that the container unit 6 is held securely within the docking portion 22. The container unit lid member 608 (not shown in Figure 3) comes packaged separately.

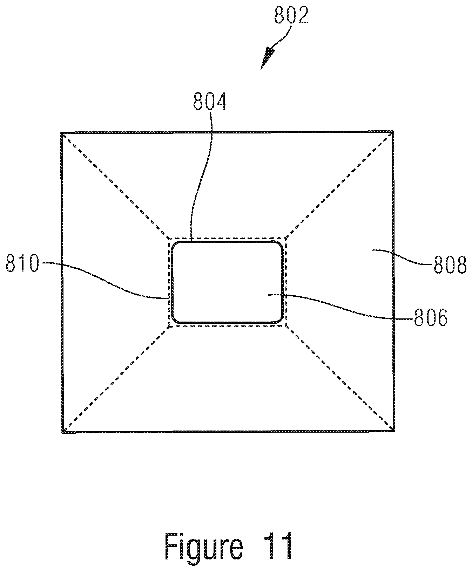

A surgical drape 802, as shown in Figure 11, is pre-packaged as a unit already secured to the container unit 6 around a peripheral edge region at a centre of the drape.

A 'surgical drape' in the context of the present disclosure is a flexible drape (typically made of cloth) that can be made sterile, or treated so that it is sterile, for use in a surgical environment to provide a sterile surface in the area of a surgical procedure and to provide a barrier against migration of bacteria.

In more detail the surgical drape 802 has a central securing portion 804 through which is provided an aperture 806, and peripheral outer portion 808 which surrounds the securing portion 804. A frangible portion 810 which comprises a perforated line extends circumferentially about the securing portion 804 thereby providing a relatively weak portion of the drape 802 between the securing portion 804 and the outer portion 808. The securing portion 804 is provided with an adhesive on an upper surface of the securing portion 804 about the periphery of the aperture 806.

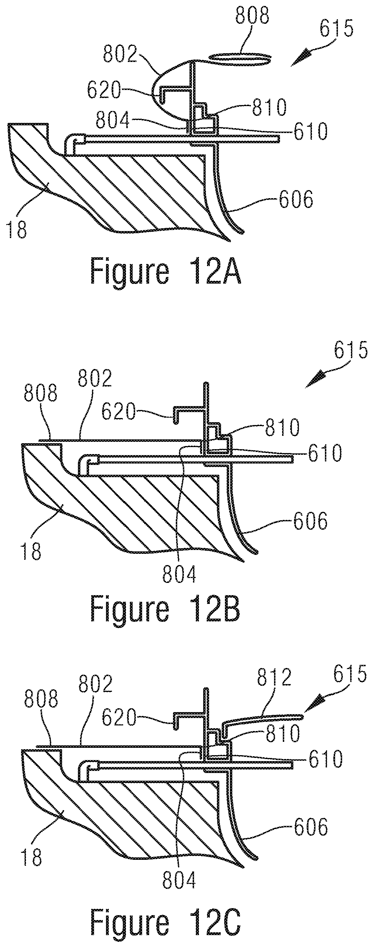

The securing portion 804 is secured to an outer surface of the outer wall 610 of the storing portion 606 of the container body 602 below the retaining lip 620, as shown in Figure 12A (note that only a portion of the surgical drape is shown attached to the outer wall on one side of the container unit and that the pipe extending through the outer wall 610 is a representation of how the fluid supply system my supply persufflating fluid to the inside of the container unit 6 an is not an accurate representation of the example shown in the previous Figures), so that the securing portion 804 surrounds the opening 615 of the storing portion 606.

Once secured, the surgical drape 802 is unfolded so that the outer portion 808 overlies the base unit 4 and so provides a sterile surface in the vicinity of the container unit 6 ensuring sterility as shown in Figure 12B. In this way a disposable container unit can be located in the non-consumable unit and the attached drape opened out away from the open mouth of the container unit provided under the lid. The drape can be spread out by a surgeon prior to location of an organ or tissue portion in the central space of the container unit. In its opened out or spread state the sterile surgical drape covers and thus protects the whole of the upper surface of the non-consumable base unit 4. The drape thus protects the user interface 48 and top and sides of the base unit. A surgeon can then duly locate a selected one body organ or body tissue position in the container unit having first duly selected a container unit having the requisite dimensions and/or shape for the organ or tissue portion concerned. For example a container unit for a liver will have a larger internal space than a container unit for a kidney.

Prior to cross-clamp, initial set-up of the system on a 'back-bench' will be performed by suitable organ retrieval support personnel.

Following cross-clamp and flushing in accordance with local protocols, the organ/tissue will be removed to the back-bench for trimming. If en bloc the organ/tissue will be dissected from any other organs/tissues removed with it and then moved to the apparatus 2. Gross dissection trimming may occur here according to surgeon preference. If not en bloc then the organ/tissue will be directly removed into the apparatus 2.

The apparatus 2 will be powered up and the organ/tissue cannulated according to defined protocols. This may be performed with any cannula according to physician preferences but will involve an airtight seal being formed between the device outlet and the inlet into the tissue; typically a significant artery or vein. Upon attachment, the system will run according to the desired automation algorithm and the surgeon will identify any significant leaks and ligate. Ligation may be according to any locally accepted practices including but not limited to tieing with a suture, stitching with a suture, vascular/tissue sealant, other means such as ligaclip/staple.

If indicated, the surgeon will ensure that no inlet is occluded by any means including but not limited to: twist in the vessel, tip occlusion due to being pressed into the vascular wall/tissue, pinching/clamping of a tube, etc.

Any amount of desired dissection/trimming of the organ/tissue in preparation for transplantation/cell isolation may be performed during system function including either upon connection or following receipt. Once the organ indicates no occlusions and has no obvious vascular leaks (indicated by rapid bubbling typically on or around a major vessel/cannulation site) and exhibits the desired outflow; the organ should be packaged for maintenance and transport within the apparatus 2.

In an alternative example, the drape 802 may be pre-secured to the container unit 6 so that it can be packaged together with the container unit 6 as a single-use sterile article.

An organ or body tissue, such as an organ or body tissue that has been extracted from a body, is surgically prepared, for example excess tissue trimmed away, and placed through the opening 615 into the storing portion 606.

The storing portion 606 is filled with a preserving fluid either before or after the organ/body tissue has been placed into the storing portion 606. An organ will typically float in the preservation fluid PF, but may be constrained by a mesh, or similar, around it avoiding direct contact with the fluid.

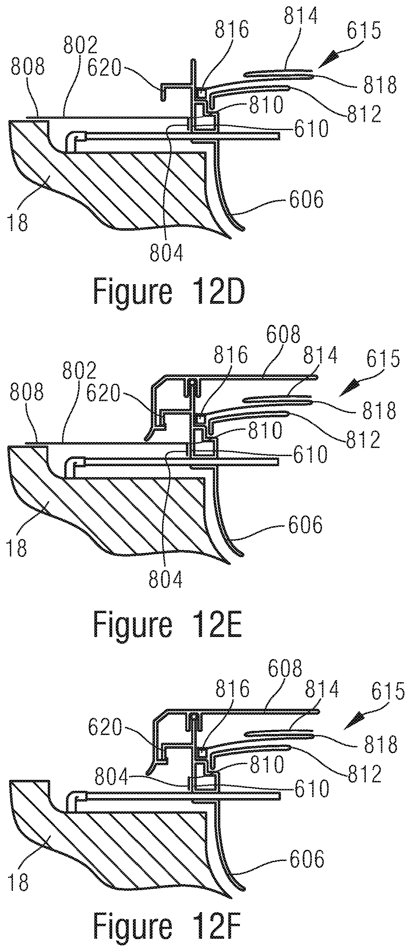

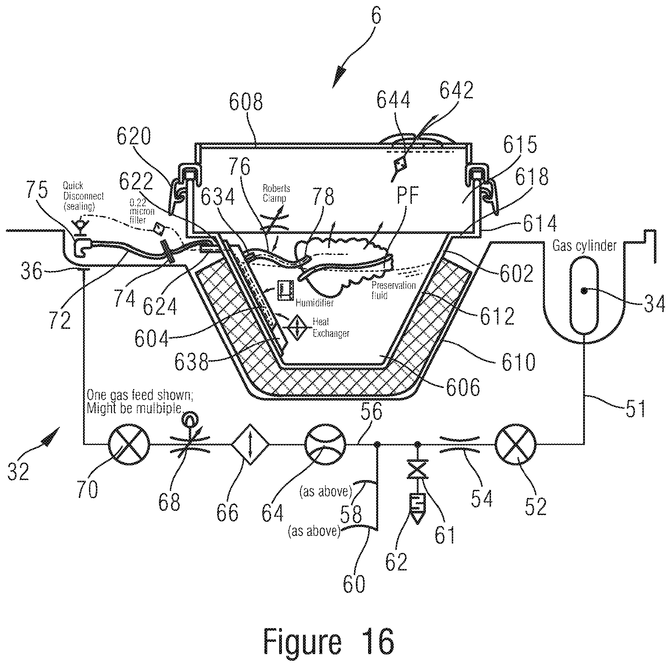

Respective cannulas are then connected to the outlet port 634 of the fluid processing device 604, as described later with reference to Figure 16, and an internal cover 812 is placed within the upper portion 614 such that it is supported by the ledge 618, as shown in Figure 12D, so as to enclose the organ/body tissue within the storing portion 606.

A second surgical drape 814 having a construction which is similar to the surgical drape 802 is provided in a folded configuration over the internal cover 812. The second surgical drape 814 has a central securing portion 816 having an adhesive on its underside and a peripheral outer portion 818. In the example shown, the second surgical drape does not have a frangible portion separating the two. The securing portion 816 is secured to an upper surface of the ledge 618, which is shown as a peripheral stepped portion of ledge in Figures 12A to 12F. Once secured, the container unit lid member 608 is secured to the retaining lip 620, thereby sealing the organ/body tissue and within the storing portion 606, as shown in Figure 12E.

The peripheral outer portion 808 of the drape 802 is then tom away from the securing portion 804 along the frangible portion 810, as shown in Figure 12F. The base unit 4 is then ready to be operated in order to start persufflation of the organ/body tissue.

Figure 16 is a schematic representation of a portion of the apparatus 2 once assembled with an organ placed within the container unit 6.

Figure 16 shows components of the fluid supply system 32 in more detail. The fluid supply system 32 comprises the fluid source 34 which is connected in series by a primary flow line 51 to a primary pressure meter 52, a primary adjustable regulator 54 in the form of a pinch valve, and first, second and third fluid supply lines 56, 58, 60 which are connected respectively to the first outlet port 36, second outlet port 38 and third outlet port 40. A vent 61 and a silencer 62 are provided between the primary adjustable regulator 54 and a junction with the first, second and third fluid supply lines 56, 58, 60. The first, second and third fluid supply lines 56, 58, 60 are arranged in parallel.

The first fluid supply line 56 comprises a flow meter 64, a heat exchanger 66, a secondary adjustable regulator 68 in the form of a pinch valve, and a secondary pressure meter 70. The second and third fluid supply lines 58, 60 are identical to the first fluid supply line 56.

A first flexible tube 72 connects the first outlet port 36 to the connector 624 of the container unit 6. The flexible tube is fitted with a filter 74 and a 'quick disconnect' connector 75. The filter is a 0.22 micron filter.

A second flexible tube 76 connects the outlet port 634 of the fluid processing device 604 to a cannula 78. It will be appreciated that as an alternative the second flexible tube 76 and cannula 78 could be provided as a single element. A flow regulator, in the form of a Roberts clamp, may fitted to the second flexible tube 76. The cannula 78 is inserted into a blood vessel of an organ or body tissue stored within the storing portion 606. The second and third fluid supply lines 58, 60 are not in use.

The container lid member 608 comprises a vent 642 which is fitted with a filter 644. The filter may be a 0.22 micron filter.

A preservation fluid PF (which may be an aqueous media) is provided within the storing portion 606. The preservation fluid may be added either before or after the organ/body tissue has been placed within the storing portion 606. In Figure 16 the storing portion 606 is filled with preservation fluid to a level which is above the second inlet port 638 and below the outlet port 634 of the fluid processing device 604. The preservation fluid PF therefore fills the internal passageway 636 of the fluid processing device 604 up to the level of the preservation fluid PF within the storing portion 606. The storing portion 606 may be prefilled with persufflation fluid PF so that it arrives sealed, sterile and ready for immediate use. The fluid processing device 604 therefore acts as a gas humidifier and heat exchanger to cool or heat and humidify persufflation gas within the fluid processing device 604 using the preservation fluid PF. It will be appreciated that as an alternative the level of the preservation fluid could be above the inlet and/or outlet port. The anatomy of the organ or tissue portion associated for use with any one container unit can determine the location of outlet port and thus a site for projection of an outwardly extending lumen designed to provide a respective gas supply for an inlet to the organ or tissue portion.

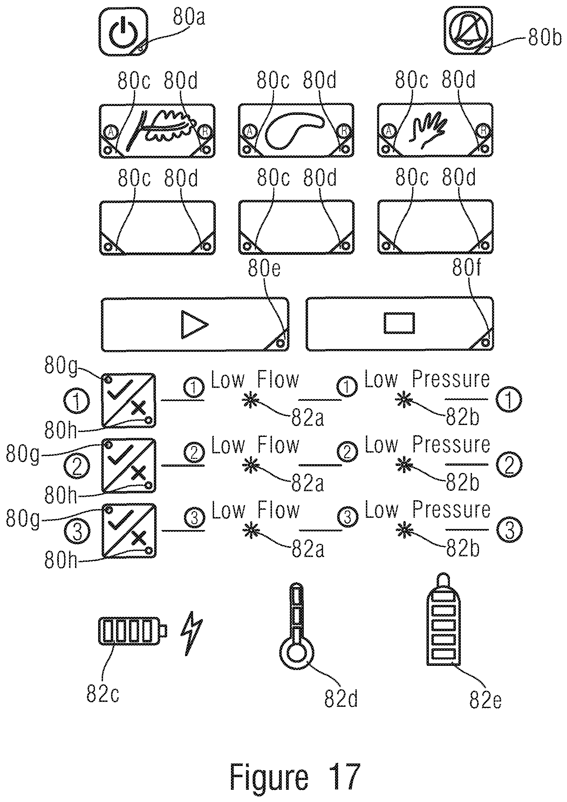

Figure 17 illustrates an example of a key pad layout for the user interface 48. The user interface 48 comprises a plurality of buttons 80a to 80h and a plurality of graphical indicators 82a - 82e. Each button 80a to 80h is a membrane button having an integral LED configured to illuminate the button 80a to 80h when actuated.

The user interface 48 comprises a standby button 80a, an alarm silencing button 80b, a plurality of organ/body tissue selection buttons 80c, 80d, an activation ('start') button 80e, a deactivation ('end') button 80f, and a plurality of line activation and deactivation buttons 80g, 80h. Alternatively the user interface (VI) can be wholly or partly provided by a touchscreen which may or may not have physical buttons on the side. It will be appreciated in the context of the present disclosure that a mobile telecommunication handset (such as a mobile phone or tablet) may be connected (e.g. via a communication interface such as a network, or via Bluetooth (RTM)). The mobile telecommunication handset may provide a user interface for the apparatus.

The standby button 80a is configured to power the control system 46 on and off. The alarm silencing button 80b is configured to deactivate or activate one or more selected alerts such as alarms. Organ/body tissue selection buttons 80c, 80d are provided for each type of organ/body tissue (for example, a pancreas, kidney or a limb) for which the apparatus 2 is to be used. In the example shown, there are first and second organ/body tissue buttons 80c, 80d are provided for each type of organ/body tissue. The first organ/body tissue button 80c is selectable for providing anterograde persufflation of the organ/body tissue and the second organ/body tissue button 80d is selectable for providing retrograde persufflation of the organ/body tissue. Blank organ/body tissue buttons 80c, 80d may be provided for future updates to the control system 46 to enable persufflation of organs having different persufflation parameters.

The activation button 80e is configured to commence flow of a persufflation fluid to the organ/body tissue stored in the apparatus 2. The deactivation button is configured to arrest flow of a persufflation fluid to the organ/body tissue stored in the apparatus 2.

A line activation button 80g and a line deactivation button 80h may be provided for activating/deactivating each fluid supply line 56, 58, 60 connected to the organ/body tissue.

The user interface 48 comprises a 'low flow' indicator 82a and a 'low pressure' indicator 82b for each fluid supply line 56, 58, 60. The user interface 48 also comprises a power level indicator 82c, a temperature indicator 82d for indicating the internal temperature within the space 22 and a pressure indicator 82e for indicating the pressure and thus remaining contents of the fluid source. Activation of these indicators may encompass a time element so as not to trigger on a single aberrant reading but for example to either trigger upon a given threshold for any of these values or to trigger if an average deviation of >30% is observed over any 3 minute period.

The base unit 4 thus provides a thermally protected area 22. Heating and/or cooling elements which may be active or passive help modulate temperature in the thermally protected area. For example, a Peltier device may be used as an active heating/cooling device. Aptly the temperature can be at least slightly heated or slightly cooled relative to ambient temperature. In this way if during the day in a hot climate temperatures in the air surrounding the base unit rise the temperature in the zone within the base unit close to the disposable container unit can be kept within an acceptable temperature range.

Aptly one or more temperature sensors are located in the climate controlled zone to provide temperature feedback to the controller. If only passive cooling or heating is used a temperature sensing feedback control can be omitted.

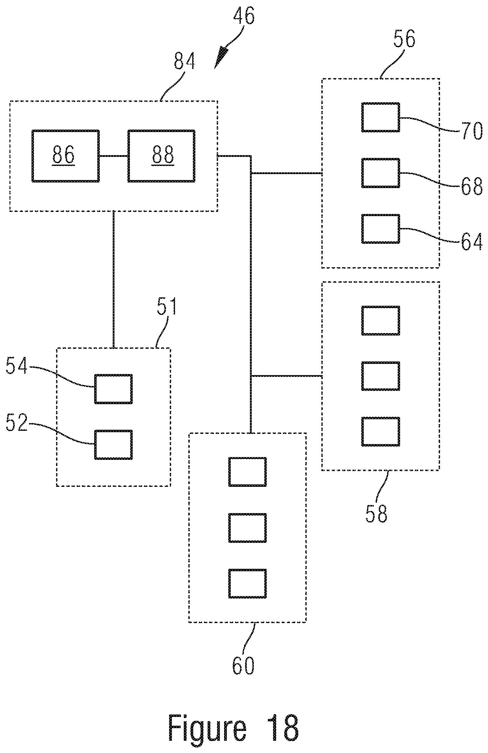

A schematic representation of the control system 46 is shown in Figure 18. The control system 46 comprises a control module 84 which includes a controller 86 and a memory 88. The controller 86 is configured to determine pressure of the fluid source 34 based on an output from the primary pressure meter 52, and to control the primary adjustable regulator 54 in accordance with the determined pressure and a predetermined pressure stored by the memory 88.

The controller 86 is further configured to determine pressure of persufflating fluid within the first fluid supply line 56 based on an output from the secondary pressure meter 70, and to control the secondary adjustable regulator 68 in accordance with said determined pressure and a further predetermined pressure stored by the memory 88. Aptly the controller can be likewise utilised to determine and control pressure and/or humidity. These can be controlled independently

The controller 86 is further configured to determine flow rate of persufflating fluid within the first fluid supply line 56 based on an output from the flow meter 64 and to control the secondary adjustable regulator 68 in accordance with said determined flow rate and a predetermined flow rate.

The controller 86 is also configured to monitor and control the flow regulating components of the second and third supply lines 58, 60 in the same manner as the first supply line 56.

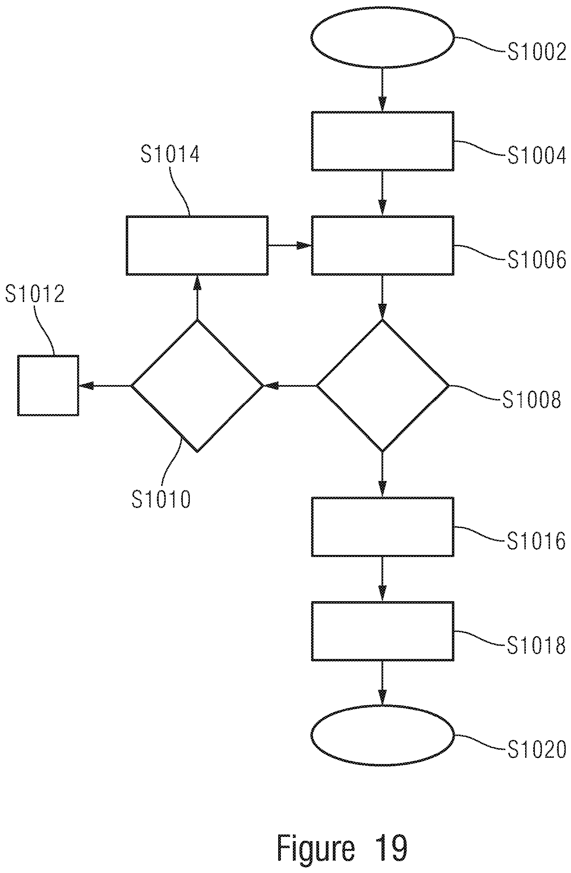

Once the organ has been connected to the fluid supply system 32 as described with reference to Figure 16, persufflation of the organ/body tissue is conducted in accordance with the method shown in Figure 19.

In step S1002, the standby button 80a is actuated in order to supply power from the power source to the remainder of the control system 46. In this step, the alarm silencing button 80b may be pressed to select whether pre-set alarms are active or inactive according to preference.

In step S1004, the appropriate organ/body tissue selection button is actuated in order to select the correct perfusion control function dependent on the type of organ to be persufflated and the type of persufflation required (i.e. anterograde or retrograde persufflation). The controller 86 is preprogramed to selectively supply persufflation fluid to the first, second and/or third supply lines 56, 58, 60 at a predetermined pressure and/or flow rate. Aptly the range of flow rate that can be provided and a desired flow rate set is 10 to 100 ml/min. The larger flow rate may for example be useful for a liver which is a relatively big aerobically active organ. Aptly the range is 15 to 30 ml/min.

It is possible as an altemative to just determine a single parameter associated with persufflation. For example for retrograde persufflation techniques/operation only pressure is controlled and flow rate is not controlled. Alternatively for anterograde persufflation flow rate alone is determined and pressure is left uncontrolled.

For example, if anterograde persufflation of a pancreas is selected, the controller may be configured to supply fluid to all three supply lines 56, 58, 60 by controlling the secondary adjustable regulator 68 of each supply line 56, 58, 60 to supply a persufflation fluid at stepped increases in pressure between 25mmHg and 60mmHg until a predetermined flow rate, for example 25 mL/min, is reached for each supply line.

If anterograde persufflation of a kidney is selected, the controller 86 may be configured to supply fluid to two of the three supply lines 56, 58, 60 in order to supply persufflating fluid to a primary renal artery and an accessory vessel by controlling the secondary adjustable regulator 68 of each supply line 56, 58, 60 to supply a persufflation fluid at stepped increases in pressure between 60mmHg and 80mmHg until a predetermined flow rate, for example 25 mL/min, is reached for each supply line.

If anterograde persufflation of a heart is selected, the controller 86 may be configured to supply fluid to supply lines 56, 58, 60 connected to each coronary artery by controlling the secondary adjustable regulator 68 of each supply line 56, 58, 60 to supply a persufflation fluid at stepped increases in pressure between 64mmHg and 85mmHg until a predetermined flow rate, for example 25 mL/min, is reached for each supply line.

If retrograde persufflation of a liver is selected, the controller may be configured to supply fluid to the desired number of supply lines 56, 58, 60 by controlling the secondary adjustable regulator 68 of each supply line 56, 58, 60 to supply a persufflation fluid at a set pressure of 18mmHg.

If retrograde persufflation of a kidney is selected, the controller may be configured to supply fluid to the desired number of supply lines 56, 58, 60 by controlling the secondary adjustable regulator 68 of each supply line 56, 58, 60 to supply a persufflation fluid at a set pressure of 18mmHg.

It is envisaged that each of the supply lines 56, 58, 60 may be controlled independently in order to supply a persufflation fluid having a predetermined pressure and/or flow rate to a particular part of an organ or body tissue to which each respective supply line 56, 58, 60 is connected.

In each of the above examples, the flow rate and pressure is determined at step S1006. It is then determined in step S1008 whether the flow rate and pressure satisfy the predetermined flow rate and the predetermined pressure. If the determined flow rate and/or the determined pressure does not satisfy the predetermined flow rate, it is then determined in step S1010 whether the determined flow rate and/or the determined pressure satisfy an alert condition. If it is determined in step S1010 that an alert condition is met, an alert is activated in step S1012 to trigger inspection for a leak or obstruction. The alert may be a visual or audible alert. If an alert condition is not met, the pressure and/or flow rate is incremented to a predetermined intermediate value or by a predetermined amount in step S1014. The necessary steps S1006 through S1014 are then repeated until the flow rate satisfies the predetermined flow rate. Aptly the pressure (or indeed one or more other parameters) is increased and/or decreased continually as needed throughout persufflation. If it is determined in step S1008 that the flow rate and pressure satisfy the predetermined flow rate and the predetermined pressure, operation proceeds to step S1016 in which the system enters a maintenance mode to supply the persufflation fluid at the predetermined flow rate and pressure.

In each instance, the temperature within the cavity within which the organ/body tissue is stored is monitored and indicated to a user via the temperature indicator 82d. If it is determined that the temperature is outside of a desired temperature ranges, such as between 4 degrees centigrade and 20 degrees centigrade, for example between 4 degrees centigrade and 8 degrees centigrade, the controller 86 activates an alert. In alternative examples comprising an active heating element and/or an active cooling element, the heating element/cooling element may be controlled to maintain the temperature of the organ/body tissue within the desired temperature range.

A persufflation fluid passes through the internal passageway 636 of the fluid processing device 604 it travels through the convoluted path provided by the baffles 640 which cause the persufflating fluid to be both cooled and humidified by the preservation fluid PF within the internal passageway 636. Preferably, the gas is humidified to saturation.

It is anticipated that that the persufflation fluid will comprises an oxygen gas mixture comprising 40% oxygen. This provides a satisfactory balance between providing satisfactory oxygenation of tissue without being toxic. Aptly the oxygen content is between 20% to 95% oxygen generally. If anterograde operation is being used the oxygen range is 30% to 40%. If retrograde operation is being used 90% to 95% oxygen is used.

Once the persufflating fluid is being supplied to the organ/body tissue at the desired flow rate and at the desired pressure, the base unit lid member 10 is then secured to the lower portion 8 ready for transport.

The control system 46 remains active throughout transportation to monitor divergences in the flow rate and pressure and control the flow rate and/or pressure in response and also to alert an operator when the pressure, temperature or flow rate of the persufflating fluid is abnormal.

Subsequent to a surgeon duly locating an organ or body tissue portion in the container and sealing it with the lid of the container unit the base unit and container unit system constantly governs itself. During this period the system constantly and automatically tries to maintain one or more parameters on each line supplying persufflation gas to a respective region of an organ or body tissue at a desired target value or within a desired range bounding a predetermined target threshold value.

The controller optionally includes a logger which stores values for monitored parameters. Aptly a time/date entry is stored along with recorded parameter (such as pressure, temperature, flow rate and/or humidity) values in step S1018. Alarm codes associated with leak or blockage alarms can also be stored. This provides helpful data for a surgeon subsequent to transport of the organ or tissue portion is likely to still be fit for purpose.

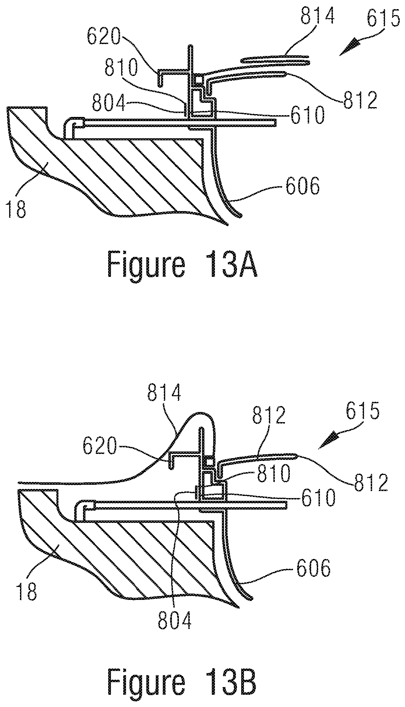

On arrival at an intended destination or after the organ/body tissue has been held in storage for a desired period of time, the base unit lid member 10 is removed. The container unit lid member 608 is also removed to reveal the second surgical drape 814, as shown in Figure 13A. The second surgical drape 814 is then unfolded so that it lies across the base unit 4 and so provides a sterile work surface, as shown in Figure 13B. The internal cover 812 is then removed and the organ/body tissue is removed from the storing portion 606 for inspection and further processing.

For example, while the system is running, the surgeon may wish to perform further dissection of the organ/body tissue in preparation for implantation/cell isolation. The organ/body tissue may then be removed from the cannula and the system shut down. This may be done according to local policy. At this point, it may be desirable to manually flush the organ/tissue with liquid/preservation solution to purge the gas from it. This may be done according to surgeon preference with a syringe, by hanging a bag, or by other means. At this point, the organ will be managed in a standardized manner for transplantation/cell isolation.

Once the apparatus 2 is not being used, it is powered off at step S1020 by pressing the standby button 80a.

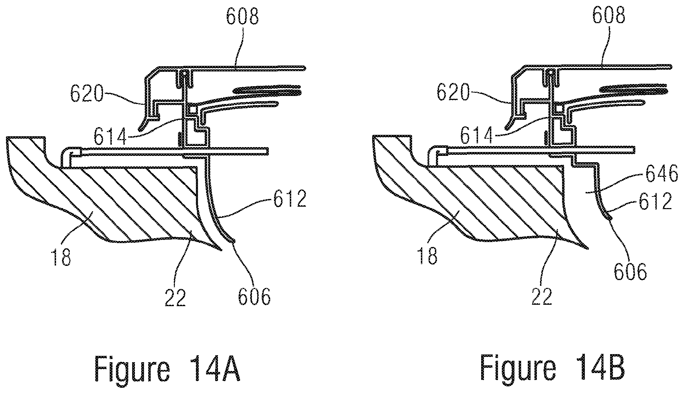

Figures 14A and 14B illustrate how the lower portion 612 of the storing portion 606 may be configured in accordance with the organ or body tissue type for which is to be used. For example, the lower portion 612 shown in Figure 14A is relatively large in capacity, whereas the lower portion 612 shown in Figure 14B is relatively small in capacity. The upper portion 614 of the storing portion 606 of both examples are, however, the same and are configured to engage with the docking portion 22 of the base unit 4. Furthermore, the space between the lower portion 612 of the arrangement shown in Figure 14B may be filled with a packing material 646 in order to accommodate a smaller capacity lower portion 612. Thus, a bespoke container unit 6 can be configured/selected for a specific type of organ/body tissue and used within the same base unit 4. Each container unit is thus to some extent bespoke to a particular type of organ or body tissue portion. Container units can optionally be colour coded or visibly marked in some way to help a health care professional select a required container unit. For example red container units for ears, green for livers, purple for kidneys white for hands and yellow for face parts.

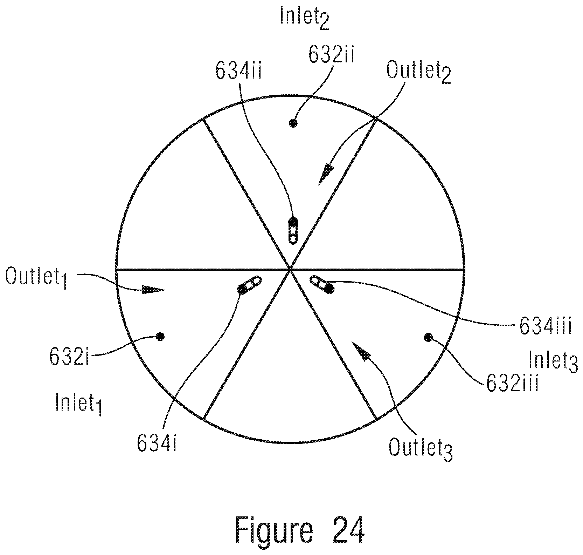

Each respective container unit not only has a respective internal dimensions/shape but also a requisite or ideal number of outlets in desired locations around the circumference of the container unit space. For example, as shown in Figure 24, a container unit may have three separate outlets 634i, 634ii, 634iii spaced around the circumference of the container unit space. The outlets may be positioned to be aligned with a vessel or an organ or body tissue having a predetermined arrangement within the container unit to aid connection between of a cannula arrangement connected to the outlet to the correct vessel.

Each outlet 634i, 634ii, 634iii may be connected to a respective inlet 632i, 632ii, 632iii which is connected, in turn, to one of the flow lines of the base unit. A schematic representation of certain components of the container unit which are connected between each of the outlets 634i, 634ii, 634iii and respective inlets 632i, 632ii, 632iii is shown in Figure 24. The components are shown for only one flow line and comprise a quick disconnect 75, 0.22 micron filter 75i, fluid processing device 604i (which comprises a heat exchanger and humidifier which are illustrated as separate components) a Roberts clamp and a connector for connecting to a cannula. Thus, a flow of persufflating gas can be supplied along each flow line at a specific flow rate and pressure which is suitable for the vessel or portion of the organ/body tissue which it supplies. This helps a surgeon efficiently connect lumens providing persufflation gas to desired respective regions of any organ or body tissue portion. Since container units may be of different size according to use a packer member or multiple packer members can be located between opposed surfaces of the container unit and the base unit.

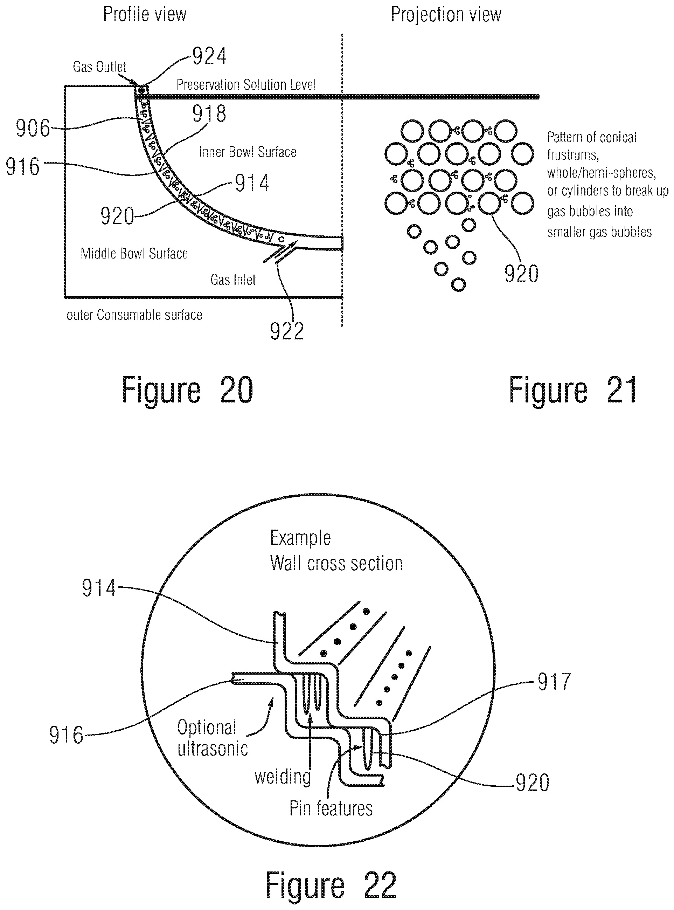

Figure 20 shows an example in which fluid processing device is formed integrally with a lower portion 912 of a storing region 906 of a container unit. The lower portion 912 comprises an inner wall 914 and an outer wall 916 between which is defined an internal flow passageway 918 having baffles 920, in the form of cylindrical, conical or hemi-spherical formations, as shown in Figure 21, within it. The internal flow passageway has a fluid inlet 922 at the lower end though which the persufflating fluid is introduced and a fluid outlet 924 at the upper end. Figure 22 shows a variation of the example shown in Figures 20 and 21 in which the inner wall 914 and the outer wall 916 are stepped and are joined together at the corners, for example by ultrasonic welding, to form flow channels 917 extending circumferentially between the walls 914, 916. The flow channels 917 may be in fluid communication with each other. The baffles 920 are formed by pin features which are formed integrally with the upper wall 914 and extend downwardly within the flow channels 917.

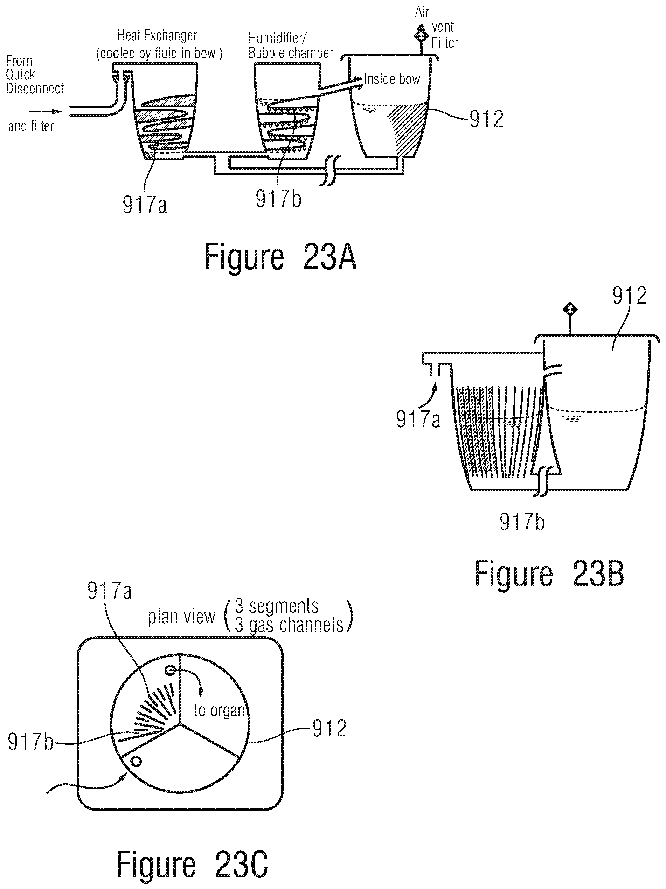

Figure 23A shows a variation of the example shown in Figures 20 and 21 in which heat exchange is conducted in a first set of channels 917a and humidification is conducted in a second set of channels 917b independently of each other. Figure 23A does not show the actual layout, but is intended to illustrate how certain components of the container unit are connected together. Both the first set of channels 917a and the second set of channels 917b are provided in fluid communication with bowl-like storage region of the container unit and are arranged in a fluted formation as shown in Figure 23B. The first and second sets of channel 917a, 917b may be provided in a segment of the container unit only, as shown in Figure 23C, and may be provided with an inlet port and an outlet port as described above. The arrangement may be replicated in other segments of the container unit for other flow channels used to persufflate and organ or body tissue. In this example, the heat exchange takes place before humidification which helps avoid a build up of condensation in the system upstream of the humidifier.

Each container unit can provide one, two, three or more persufflation gas outlets connectable via a respective lumen to a respective location of a specific organ or body tissue portion. Each outlet is supplied with gas having tailored qualities specific to the location on the organ or body tissue portion to which that gas is to be supplied. Thus each channel is independently parameter controllable. For example each channel can have a different (or optionally the same) temperature and/or pressure and/or flow rate and/or humidity and/or composition. These can be varied over time to fit a desired parameter profile. This is convenient when different regions of an organ or body tissue portion have different physical characteristics (such as density, porosity or granularity or the like).

Aptly the retained unit or base unit (e.g. the storage apparatus) is usage neutral whilst the disposable unit is organ type or body tissue portion type specific.

It will be appreciated that the container unit could be configured for use with the base unit 4 described in the previous examples.

It is anticipated that certain examples of the apparatus may be suitable for multiple organ/ partial organ / composite tissue types (e.g. pancreas, kidney, liver, heart, bowel, lung, hand, arm, foot, leg, face, skin, etc.); anterograde or retrograde; for transplantation or cell isolation or other R&D applications. In particular, it is anticipated that certain examples of the apparatus may be suitable for anterograde persufflation of a pancreas for islet isolation.

Dedicated gas out-feed lines may be provided for exhausting gas directly through cannulas inserted into the organ or body tissue.

As described above, primary control is flow rate, secondary control is pressure, lines can be managed / controlled in series or parallel, progressively or all at same time; in an example, all lines managed at same time, in parallel, and any lines can be toggled between 'active' and inactive' automatically or manually (for example, if an apparent leak, then automatically closed after a period to prevent ongoing gas wastage, but user can resolve the leak and switch the line 'active' again without affecting other lines).

Separate leak modes and maintenance (i.e. in-use) modes may be provided. Alternatively, they can be combined into a single mode.

Possible modes of operation include:
Disposable tubing not connected; in envisaged example, when first 'started', gas will come out this port, and control system might alert user to this as a leak or similar, but after a time of no improvement might make the line 'inactive' and close it off (principally to stop gas wastage).

Disposable tubing connected, but (Roberts) clamp closes the line; in envisaged example,
this will restrict all gas flow in this line, and the control system will increase pressure to line (in case just a cannulation blockage / poor vascularisation), but after a time of no improvement might make the line 'inactive'.

Disposable tubing connected, (Roberts) clamp open, but tubing not used to cannulate organ port / vessel; in envisaged example, when first 'started', gas will come out this port, and control system might alert user to this as a leak or similar, but after a time of no improvement might make the line 'inactive' and close it off (principally to stop gas wastage).

Disposable tubing connected, (Roberts) clamp open, organ poorly cannulated, which might exhibit as either a leak or a restriction - leak or restriction modes as described above may be implemented.

Disposable tubing connected, (Roberts) clamp open, organ suitably cannulated; in envisaged example, under control system, pressure gradually increased until target flow achieved, then pressure regulated to maintain target flow.

Table 1 below illustrated possible conditions and operation modes:

**Table 1.**

| | **Pressure lower than expected** | | **Pressure OK** | | **Pressure higher than expect** | |
|---|---|---|---|---|---|---|
| **Flow too high** | Potential scenarios: | | Potential scenarios: | | Potential scenarios: | |
| | | • Tubing line open, but not used in cannulation | | • Slight leak in cannulation | | • Control system not yet reached target |
| | | • Leak in cannulation | Therefore: | | Therefore: | |
| | Therefore: | | | • Alert user to 'High Flow' via LED and remote interface | | • Maintain and manage under autoregulation control algorithm |
| | | • Alert user to 'High Flow' via LED and remote interface | | | | |
| | | • After 30 mins, switch off line to preserve gas, but LED stays lit | | • Maintain and manage under autoregulation control algorithm | | |
| | | • User can restart at any time, which would switch of the LED for a short period (but it will light again if situation remains.) | | | | |
| **Flow OK** | Potential scenarios: | | Potential scenarios: | | Potential scenarios: | |
| | | • Slightly atypical organ, but everything is OK | | • Everything is OK | | • Slightly atypical organ, but everything is OK |
| | | | Therefore: | | | |
| | Therefore: | | | • Maintain and manage under autoregulation control algorithm | Therefore: | |
| | | • Maintain and manage under autoregulation control algorithm | | | | • Maintain and manage under autoregulation control algorithm |
| **Flow too low** | Potential scenarios: | | Potential scenarios: | | Potential scenarios: | |
| | | • Control system not yet reached target | | • Tubing line clamped | | • Tubing line clamped |
| | | | | • Poor vascularisation | | • Poor vascularisation |
| | Therefore: | | Therefore: | | Therefore: | |
| | | • Maintain and manage under autoregulation control algorithm | | • Alert user to 'Low Flow' via LED and remote interface | | • Alert user to 'Low Flow' via LED and remote interface |
| | | | | • Maintain and manage under autoregulation control algorithm | | • Maintain and manage under autoregulation control algorithm |
| | | | | • No point switching off as not wasting any gas | | • No point switching off as not wasting any gas within autoregulation parameters |

Preferably, the apparatus 2 should weigh less than 20kg in total when in use.

The retained unit consists of majority of high value, high cost, reusable components and structural integrity to support the container unit (e.g. the Primary Organ Container).

The retained unit enables users / retrieval team to identify which organ they wish to persufflate (e.g. pancreas) and in which manner (e.g. anterograde), and provides ongoing monitoring and feedback of the gas in-feed lines and critical functions on the system; in one example, only critical control and feedback is via retained unit (e.g. which organ and which manner (or if under remote PC control for R&D), low pressure or low flow in individual lines, low battery, low pressure in gas source, temperature excursions), and specific values and historical charts are displayed through a remote monitoring connection (e.g. smartphone or tablet, via Bluetooth (RTM) broadcast, but no control).

The Primary Organ Container directly contains the organ and support media, could be single- use or multi-use (cleanable) or refurbishable (e.g. cleanable and replace gas lines), for one type of organ / tissue or multiple types; in preferred example, it will be to be sterile and disposable after use, principally moulded from a low cost plastic, to minimise costs and ensure clean and sterile.

The Primary Organ Container may a sealed container (with passive air vents) to prevent pressure build up during persufflation. To prevent contamination of organ/preservation media, all gas passages in and out of container (whether used or un-used) will have a filter for filtering out pathogens (e.g. to inhibit infection), such as a 0.22 micron filter on them, or a more restrictive filter, such as a 0.21 micron filter.

The Primary Organ Container may be configured to be suitable for only one organ type, some might be suitable for multiple organ types; in preferred example, focus is to minimise the amount of excess 'free' volume inside container, as this needs to be filled with a costly fluid, but external form will be consistent across all container types, to minimise free space in sealed retainer unit (as per illustrations already provided).

The Primary Organ Container may be supplied with recommended cannula, might be exact number of same size, or different interchangeable sizes according to organ variation; in preferred example, users will be able to select cannula that they think are most appropriate for organ case, including using their own preferred cannula which may not be supplied with the POC.

Cannula can connect to gas in-feed lines either above or below the fluid level; in one example, likely to be above the fluid level, which should ensure most consistent with current surgical practice.

Cannula ports can be in a regular or irregular pattern (according to organ-specific anatomical requirements), in any direction, depending on organ type; in envisaged example, likely to be an irregular pattern for pancreas.

Cannulas can stiff or flexible without occlusion; in envisaged example, likely to be flexible without occlusion.

In the secondary usage case where a secondary surgical drape is added, the adhesive region could have a tape which can be peeled off and would be placed onto an inner lip inside the organ container. In this case, the drape could either be peeled off if a soft adhesive is used or a similar perforation pattern could be implemented with repeated uses applied atop one another. If a bigger drape is needed the individual points could be folded atop one another ala an accordion. If required fold lines could be extrapolated for this; though the order of the folds is key to achieve the desired result.

Examples of modes of operation of a device in accordance with, or a variant of, the apparatus 2 described above are as follows:
An organ is attached to multiple channels with a defined flow rate maintained to each channel for the duration of leak-testing and subsequent persufflation. Flow will be directionally physiological (ie entering from and artery/portal vein and exiting a vein). Pressure thresholds will be tissue-specific but the general technique should be quite similar. A variety of parameters should be semi-continuously monitored throughout the duration of persufflation such that an end-user can monitor them remotely and a record of the procedure can be offloaded upon completion for patient records. These parameters should include but not be limited to: temperature, pressure to each line, flow rate to each line, recording of any alarms/overrides.

### 1. Turn on the device

The device is activated once the consumable (e.g. container unit) is inserted, e.g. the user should activate the device by pressing a switch.

### 2. Define a usage case

The type of organ to be perfused should be inputted to define a set of parameters for the device to operate under. These parameters/thresholds may vary for each organ or tissue to be persufflated. This may come via a user inputted code, menu, hard switch, by something internal to the consumable to avoid user error, or by some other means.

### 3. Perform system check

The system should perform a pre-use screen of all key system parameters to ensure it is capable of performing a defined persufflation 'run'. Critically, testing the remaining battery charge should be performed and if below a given threshold the user should be prompted. This may trigger basic maintenance such as plugging the device in/swapping a battery or performing other key basic maintenance steps to ensure the device is ready to use.

### 4. Define channels in use

As the device may have 4 channels to send flow to and not all will be in use for a given case, the user may need to define which channels will be utilized for the procedure. This will happen once all vessels have been cannulated but prior to beginning flow to the organ/tissue. This number can range from 1 to 3 with capability to 'turn off any channels not in use. This could be achieved by a series of Y/N questions, by a hard switch, or by entering a special mode where the user can depress a button to activate a given channel. Once this is established, a defined series of steps should have to be taken to modify the channels in use such that channels are not inadvertently activated/deactivated during use.

### 5. Enter 'leak-testing' mode

The device will now begin flowing gas to each of the channels defined above. Pressures will slowly ramp up according to a feedback loop defined below:

### 5.1 Gradually ramp pressures to each channel

The pressure/flow to each channel will slowly ramp up according to a pre-defined profile.

### 5.2 Restrict pressure increase once a target flow rate has been achieved

Once a target flow rate has been achieved the pressure will be maintained for a given channel. This operates under a 'flow-control' paradigm where we are aiming for a target flow rate while trying to minimize the pressure to a flow path avoiding any unnecessary barro-trauma. This flow rate should be continuously maintained for the rest of the procedure until the organ is disconnected or an upper pressure threshold has been reached (see below). In the case of anterograde persufflation of the pancreas we may define this target as 25 ml/min of gas flow.

### 5.3 Activate a 'user-alarm' if a channel pressure does not reach a specified threshold (low)

If the pressure does not meet a defined threshold to a given channel, the flow to the channel should be capped and the presence of a 'possible leak' should be indicated to the user. This may come in the form of a light, screen cue audible noise or other alarm. For the pancreas, this would typically occur below 10-15 mmHg but occasionally stable flows could be achieved in the 12-15 mmHg range with no observable leaks found. Therefore perhaps there could be a coded statement to this alarm (ie red for very low and yellow for low) and a user override could be built into the system should the pressure not meet this threshold in the absence of observable leaks.

### 5.4 Activate a 'user-alarm' if a channel pressure exceeds a specified threshold

If the pressure exceeds a defined threshold to a given channel, the pressure to the channel should be capped and a user alarm initiated. This should inform the user to check for an obstruction to flow through the organ or tissue. This may typically include: kinks in the tubing, twists in the vessel, or if the cannulae is somehow jammed into the tissue or vessel wall. Once the user has inspected the designated line, there should be an override function to expand the defined threshold beyond that typical for the usage case. Once a secondary upper limit has been reached, a secondary user override should enable further pressure increase to the maximum acceptable pressure for a designated usage case.

For example, in the pancreas we would expect a pressure between 15-25 mmHg to any given vascular bed. In some cases, particularly poorly flushed tissue such as that obtained following a Donation after Circulatory Death (DCD) procurement, the pressure may exceed this threshold. If this is the case we would inspect for obstructions to flow and then increase pressures to a 50 mmHg threshold. If this is reached and there are still no signs of occlusion, we may then continue to increase the pressure to an upper limit of 80 mmHg.

### 5.5 Exit leak-testing mode and enter 'maintenance mode'

Once all active channels meet predetermined guidelines and the user is happy there are no large leaks, the user shall exit the 'leak testing mode' and proceed to maintenance mode. This will restrict the capacity to alter active channels and aim to maintain the specified flow to each channel in use.

### 6. Maintenance mode

Should the pressure dramatically increase to maintain a given flow > 10 mmHg from entering maintenance mode, an alarm should be triggered indicating an obstruction to flow.

If facilities are available, the user should then have the capacity to re-enter 'leak-testing mode' to fix the problem.

Typically, as the vascular bed relaxes and is cleared of liquid, the pressure required to maintain a given flow rate drops 5-10 mmHg over the first 30-60 min of persufflation. This has been observed across multiple organs/tissues and should be accounted for such that the flow is limited to the target value; minimizing the opportunity for barrotrauma.

### 'Series A-PSF'

An organ is attached to multiple channels with a defined flow rate maintained to each channel for the duration of leak-testing and subsequent persufflation. Flow will be directionally physiological (ie entering from and artery/portal vein and exiting a vein). Pressure thresholds will be tissue-specific but the general technique should be quite similar. A variety of parameters should be semi-continuously monitored throughout the duration of persufflation such that an end-user can monitor them remotely and a record of the procedure can be offloaded upon completion for patient records. These parameters should include but not be limited to: temperature, pressure to each line, flow rate to each line, recording of any alarms/overrides.

### 1. Turn on the device

The user should activate the device by pressing a switch.

### 2. Define a usage case

The type of organ to be perfused should be inputted to define a set of parameters for the device to operate under. These parameters/thresholds may vary for each organ or tissue to be persufflated. This may come via a user inputted code, menu, hard switch, by something internal to the consumable to avoid user error, or by some other means.

### 3. Perform system check

The system should perform a pre-use screen of all key system parameters to ensure it is capable of performing a defined persufflation 'run'. Critically, testing the remaining battery charge should be performed and if below a given threshold the user should be prompted. This may trigger basic maintenance such as plugging the device in/swapping a battery or performing other key basic maintenance steps to ensure the device is ready to use.

### 4. Define channels in use

As the device will have 4 channels to send flow to and not all will be in use for a given case, the user will need to define which channels will be utilized for the procedure. This will happen once all vessels have been cannulated but prior to beginning flow to the organ/tissue. This number can range from 1 to 3 with capability to 'turn off any channels not in use. This could be achieved by a series of Y/N questions, by a hard switch, or by entering a special mode where the user can depress a button to activate a given channel. Once this is established, a defined series of steps should have to be taken to modify the channels in use such that channels are not inadvertently activated/deactivated during use.

### 5. Enter 'leak-testing' mode

The device will now begin flowing gas to each of the channels defined above. Pressures will slowly ramp up according to a feedback loop defined below:

### 5.1 Gradually ramp pressures to the selected channel

The pressure/flow to each channel will slowly ramp up according to a pre-defined profile.

### 5.2 Restrict pressure increase once a target flow rate has been achieved

Once a target flow rate has been achieved the pressure will be maintained to the channel. This operates under a 'flow-control' paradigm where we are aiming for a target flow rate while trying to minimize the pressure to a flow path avoiding any unnecessary barro-trauma. This flow rate should be continuously maintained until testing of this channel has been completed an upper pressure threshold has been reached (see below). In the case of the pancreas we have defined this target as 25 ml/min of gas flow.

### 5.3 Activate a 'user-alarm' if a channel pressure does not reach a specified threshold (low)

If the pressure does not meet a defined threshold, the presence of a 'possible leak' should be indicated to the user. This may come in the form of a light, screen cue audible noise or other alarm. For the pancreas, this would typically occur below 10-15 mmHg but occasionally stable flows could be achieved in the 12-15 mmHg range with no observable leaks found. Therefore perhaps there could be a coded statement to this alarm (ie red for very low and yellow for low) and a user override could be built into the system should the pressure not meet this threshold in the absence of observable leaks. Alarm may also be embodied by a low frequency audible noise (beep).

### 5.4 Activate a 'user-alarm' if a channel pressure exceeds a specified threshold

If the pressure exceeds a defined, the pressure to the channel should be capped and a user alarm initiated. This should inform the user to check for an obstruction to flow through the organ or tissue. This may typically include: kinks in the tubing, twists in the vessel, or if the cannulae is somehow jammed into the tissue or vessel wall. Once the user has inspected the line, there should be an override function to expand the defined threshold beyond that typical for the usage case. Once a secondary upper limit has been reached, a secondary user override should enable further pressure increase to the maximum acceptable pressure for a designated usage case. Alarm may also be embodied by a high frequency audible noise (beep).

For example, in the pancreas we would expect a pressure between 15-25 mmHg to any given vascular bed. In some cases, particularly poorly flushed tissue such as that obtained following a DCD procurement, the pressure may exceed this threshold. If this is the case we would inspect for obstructions to flow and then increase pressures to a 50 mmHg threshold. If this is reached and there are still no signs of occlusion, we may then continue to increase the pressure to an upper limit of 80 mmHg.

### 5.5 Proceed to inspection of the next channel

Once the user is happy with a channel, the flows to this channel can be stopped and the process repeated (steps 5.1-5.5) for the next channel. If there are no further channels to leak- test, the user will indicate this to the device and flow will be restored to all channels.

### 5.6 Exit leak-testing mode and enter 'maintenance mode'

Once all active channels meet predetermined guidelines and the user is happy there are no large leaks, the user shall exit the 'leak testing mode' and proceed to maintenance mode. This will restrict the capacity to alter active channels and aim to maintain the specified flow to each channel in use.

### 6. Maintenance mode

Should a sharp decline in pressure to any channel occur; such as greater than 15 mmHg over any 3 min period, an alarm should be triggered to inspect the line with a timestamp of when this occurred. Likewise, should the pressure dramatically increase to maintain a given flow >10 mmHg from entering maintenance mode, an alarm should be triggered indicating an obstruction to flow.

If facilities are available, the user should then have the capacity to re-enter 'leak-testing mode' to fix the problem.

Typically, as the vascular bed relaxes and is cleared of liquid, the pressure required to maintain a given flow rate drops 5-10 mmHg over the first 30-60 min of persufflation. This has been observed across multiple organs/tissues and should be accounted for such that the flow is limited to the target value; minimizing the opportunity for barrotrauma.

### 'R-PSF'

For this case, flow will run directionally opposite of physiological blood flow. For example, flow may enter from a vein and exit either through the artery or through a series of 'pin pricks' manually placed into the organ to act as a drainage shunt. Pressure thresholds will be tissue- specific but the general technique should be quite similar. A variety of parameters should be semi-continuously monitored throughout the duration of persufflation such that an end-user can monitor them remotely and a record of the procedure can be offloaded upon completion for patient records. These parameters should include but not be limited to: temperature, pressure to each line, flow rate to each line, recording of any alarms/overrides.

### 1. Turn on the device

The user should activate the device by pressing a switch.

### 2. Define a usage case

The type of organ to be perfused should be inputted to define a set of parameters for the device to operate under. These parameters/thresholds may vary for each organ or tissue to be persufflated. This may come via a user inputted code, menu, hard switch, by something internal to the consumable to avoid user error, or by some other means.

### 3. Perform system check

The system should perform a pre-use screen of all key system parameters to ensure it is capable of performing a defined persufflation 'run'. Critically, testing the remaining battery charge should be performed and if below a given threshold the user should be prompted. This may trigger basic maintenance such as plugging the device in/swapping a battery or performing other key basic maintenance steps to ensure the device is ready to use.

### 4. Define channels in use

As the device will have 4 channels to send flow to and not all will be in use for a given case, the user will need to define which channels will be utilized for the procedure. This will happen once all vessels have been cannulated but prior to beginning flow to the organ/tissue. This number can range from 1 to 3 with capability to 'turn off' any channels not in use. This could be achieved by a series of Y/N questions, by a hard switch, or by entering a special mode where the user can depress a button to activate a given channel. Once this is established, a defined series of steps should have to be taken to modify the channels in use such that channels are not inadvertently activated/deactivated during use.

### 5. Maintenance mode

As R-PSF operates on a 'pressure controlled' paradigm, the system will need to supply a constant pre-defined pressure to a given channel for the duration of the procedure. For example, for the liver this may be set to 18 mmHg. Flow parameters should be monitored and recorded until cessation of persufflation.

In the drawings like reference numerals refer to like parts. It will be appreciated from the discussion above that the embodiments shown in the figures are merely exemplary, and include features which may be generalised, removed or replaced. The scope of the invention is defined in the appended claims. With reference to the drawings in general, it will be appreciated that schematic functional block diagrams are used to indicate functionality of systems and apparatus described herein. In addition the processing functionality may also be provided by devices which are supported by an electronic device. It will be appreciated however that the functionality need not be divided in this way, and should not be taken to imply any particular structure of hardware other than that described. The function of one or more of the elements shown in the drawings may be further subdivided, and/or distributed throughout apparatus of the disclosure. In some embodiments the function of one or more elements shown in the drawings may be integrated into a single functional unit.

As will be appreciated by the skilled reader in the context of the present disclosure, each of the examples described herein may be implemented in a variety of different ways.

Certain features of the methods described herein may be implemented in hardware, and one or more functions of the apparatus may be implemented in method steps. It will also be appreciated in the context of the present disclosure that the methods described herein need not be performed in the order in which they are described, nor necessarily in the order in which they are depicted in the drawings. Accordingly, aspects of the disclosure which are described with reference to products or apparatus are also intended to be implemented as methods and vice versa. The methods described herein may be implemented in computer programs, or in hardware or in any combination thereof. Computer programs include software, middleware, firmware, and any combination thereof. Such programs may be provided as signals or network messages and may be recorded on computer readable media such as tangible computer readable media which may store the computer programs in non-transitory form. Hardware includes computers, handheld devices, programmable processors, general purpose processors, application specific integrated circuits (ASICs), field programmable gate arrays (FPGAs), and arrays of logic gates.

Other examples and variations of the disclosure will be apparent to the skilled addressee in the context of the present disclosure. The scope of the invention is defined in the claims.

## Claims

1. An apparatus for storing an organ or body tissue, the apparatus comprising:
a container unit (6) comprising a container body (602) defining a storage region (606) for storing an organ or body tissue, wherein the container body (602) includes an opening (615) to enable the organ or body tissue to be admitted to, and removed from, the storage region (606) through the opening (615); and
a surgical drape (802) comprising: (i) a securing portion (804) which is securable to the container unit (6), (ii) a peripheral outer portion (808) which surrounds the securing portion (804), and (iii) a frangible portion (810) separating the securing portion (804) from the peripheral outer portion (808);
wherein the securing portion (804) is securable to the container body (602) to enable the drape (802) to extend around a portion of the periphery of the opening (615) with the peripheral outer portion (808) of the drape (802) arranged outward of the opening (615); and
wherein the frangible portion (810) extends around the securing portion (804) to enable the peripheral outer portion (808) to be separated from the securing portion (804) by tearing the frangible portion (810).

2. The apparatus of claim 1, wherein the container unit (6) has an attachment portion to which the surgical drape (802) is securable.

3. The apparatus of claim 2, wherein the attachment portion surrounds at least a portion of the opening (615) of the container unit (6).

4. The apparatus of any preceding claim, wherein the securing portion (804) of the drape (802) comprises an adhesive region for securing the drape (802) to the container unit (6).

5. The apparatus of any preceding claim, wherein the drape (802) includes an access opening which corresponds to the opening (615) of the container unit (6) to enable the surgical drape (802) to be secured to the container unit (6) with the access opening in alignment with the opening (615) of the container unit (6).

6. The apparatus of claim 5, as dependent on claim 4, wherein the adhesive region surrounds at least a portion of the access opening.

7. The apparatus of any preceding claim, wherein the surgical drape (802) comprises a first surgical drape, and the apparatus further comprises a second surgical drape; and
wherein the second surgical drape comprises a second securing portion which is securable to the container body (602) to enable the second drape to extend around a portion of the periphery of the opening (615).

8. The apparatus of claim 7, wherein the container unit (6) comprises a second attachment portion to which the second surgical drape is securable.

9. The apparatus of claim 8, wherein the second attachment portion comprises an inner surface of a wall of the container body.

10. The apparatus of any preceding claim, wherein the container unit (6) further comprises an inner lid member which is configured to be secured to the container body (602) so as to enclose the organ or body tissue within the storage region (606) between the container body (602) and the inner lid member, optionally wherein the container unit (6) comprises an outer lid member which is configured to be secured to the container body (602) so as to enclose the inner lid member between the container body (602) and the outer lid member.

11. The apparatus of claim 10, as dependent on claim 7, or any claim dependent thereon, wherein the apparatus is arranged to enable the second drape to be secured to the container body (602) so that a portion of the second drape overlies the inner lid member.

12. The apparatus of any preceding claim, further comprising a storage apparatus (2) comprising: (i) a fluid supply system comprising a store of persufflation fluid to be supplied to a said organ or body tissue, and (ii) a container unit receiving portion;
wherein the container unit (6) is removably insertable into the container unit receiving portion of the storage apparatus (2); and
wherein the securing portion (804) of the drape (802) is securable to the container body (602) to enable the drape (802) to extend around the portion of the periphery of the opening (615) with the peripheral outer portion (808) of the drape (802) arranged outward of the opening (615) to protect a portion of a surface of the storage apparatus (602).

13. The apparatus of claim 12, wherein the portion of the surface of the storage apparatus (2) comprises a portion of a periphery of the container unit receiving portion.

14. The apparatus of any of claims 1 to 13, wherein the securing portion (804) is secured to the container body (602) so that the drape (802) extends around the portion of the periphery of the opening (615) with the peripheral outer portion (808) of the drape (802) arranged outward of the opening (615).

15. A method of providing an organ or body tissue in an apparatus for storing an organ or body tissue as defined in any preceding claim, wherein the method comprises:
placing the organ or body tissue through the opening (615) in the container unit (6) and into the storage region (606) of the container unit (6); and
tearing along the frangible portion (810) of the drape (802) to remove the peripheral outer portion (808) of the drape (802) after the organ or body tissue has been placed into the storage region (606) of the container unit (6).

## Patentansprüche

1. Einrichtung zum Lagern eines Organs oder Körpergewebes, wobei die Einrichtung Folgendes umfasst:
eine Behältereinheit (6), die einen Behälterkörper (602) umfasst, der einen Lagerungsbereich (606) zum Lagern eines Organs oder Körpergewebes definiert, wobei der Behälterkörper (602) eine Öffnung (615) enthält, um zu ermöglichen, dass das Organ oder Körpergewebe durch die Öffnung (615) in den Lagerungsbereich (606) eingeführt und aus diesem entnommen wird; und
ein OP-Tuch (802), das Folgendes umfasst: (i) einen Sicherungsabschnitt (804), der an der Behältereinheit (6) gesichert werden kann, (ii) einen äußeren Umfangsabschnitt (808), der den Sicherungsabschnitt (804) umschließt, und (iii) einen brüchigen Abschnitt (810), der den Sicherungsabschnitt (804) von dem äußeren Umfangsabschnitt (808) trennt;
wobei der Sicherungsabschnitt (804) an dem Behälterkörper (602) gesichert werden kann, um es dem Tuch (802) zu ermöglichen, sich um einen Abschnitt des Umfangs der Öffnung (615) zu erstrecken, wobei der äußere Umfangsabschnitt (808) des Tuchs (802) außerhalb der Öffnung (615) angeordnet ist; und
wobei sich der brüchige Abschnitt (810) um den Sicherungsabschnitt (804) erstreckt, um zu ermöglichen, dass der äußere Umfangsabschnitt (808) durch Reißen des brüchigen Abschnitts (810) von dem Sicherungsabschnitt (804) getrennt wird.

2. Einrichtung nach Anspruch 1, wobei die Behältereinheit (6) einen Befestigungsabschnitt aufweist, an dem das OP-Tuch (802) gesichert werden kann.

3. Einrichtung nach Anspruch 2, wobei der Befestigungsabschnitt wenigstens einen Abschnitt der Öffnung (615) der Behältereinheit (6) umschließt.

4. Einrichtung nach einem vorhergehenden Anspruch, wobei der Sicherungsabschnitt (804) des Tuchs (802) einen Klebebereich zum Sichern des Tuchs (802) an der Behältereinheit (6) umfasst.

5. Einrichtung nach einem vorhergehenden Anspruch, wobei das Tuch (802) eine Zugangsöffnung beinhaltet, die der Öffnung (615) der Behältereinheit (6) entspricht, um zu ermöglichen, dass das OP-Tuch (802) an der Behältereinheit (6) gesichert wird, wobei die Zugangsöffnung an der Öffnung (615) der Behältereinheit (6) ausgerichtet ist.

6. Einrichtung nach Anspruch 5, wie von Anspruch 4 abhängig, wobei der Klebebereich wenigstens einen Abschnitt der Zugangsöffnung umschließt.

7. Einrichtung nach einem vorhergehenden Anspruch, wobei das OP-Tuch (802) ein erstes OP-Tuch umfasst und die Einrichtung ferner ein zweites OP-Tuch umfasst; und
wobei das zweite OP-Tuch einen zweiten Sicherungsabschnitt umfasst, der an dem Behälterkörper (602) gesichert werden kann, um zu ermöglichen, dass sich das zweite Tuch um einen Abschnitt des Umfangs der Öffnung (615) erstreckt.

8. Einrichtung nach Anspruch 7, wobei die Behältereinheit (6) einen zweiten Befestigungsabschnitt umfasst, an dem das zweite OP-Tuch gesichert werden kann.

9. Einrichtung nach Anspruch 8, wobei der zweite Befestigungsabschnitt eine innere Oberfläche einer Wand des Behälterkörpers umfasst.

10. Einrichtung nach einem vorhergehenden Anspruch, wobei die Behältereinheit (6) ferner ein inneres Deckelelement umfasst, das dazu konfiguriert ist, an dem Behälterkörper (602) gesichert zu werden, um das Organ oder Körpergewebe innerhalb des Lagerungsbereichs (606) zwischen dem Behälterkörper (602) und dem inneren Deckelelement einzuschließen, wobei die Behältereinheit (6) optional ein äußeres Deckelelement umfasst, das dazu konfiguriert ist, an dem Behälterkörper (602) gesichert zu werden, um das innere Deckelelement zwischen dem Behälterkörper (602) und dem äußeren Deckelelement einzuschließen.

11. Einrichtung nach Anspruch 10, wie von Anspruch 7 abhängig, oder einem davon abhängigen Anspruch, wobei die Einrichtung angeordnet ist, um zu ermöglichen, dass das zweite Tuch an dem Behälterkörper (602) gesichert wird, sodass ein Abschnitt des zweiten Tuchs das innere Deckelelement überdeckt.

12. Einrichtung nach einem vorhergehenden Anspruch, das ferner eine Lagerungseinrichtung (2) umfasst, die Folgendes umfasst: (i) ein Flüssigkeitszufuhrsystem, das einen Vorrat an Persufflationsflüssigkeit umfasst, der an ein Organ oder Körpergewebe zugeführt werden soll, und (ii) einen Aufnahmeabschnitt der Behältereinheit;
wobei die Behältereinheit (6) entfernbar in den Aufnahmeabschnitt der Behältereinheit der Lagerungseinrichtung (2) eingesetzt werden kann; und
wobei der Sicherungsabschnitt (804) des Tuchs (802) an dem Behälterkörper (602) gesichert werden kann, um es dem Tuch (802) zu ermöglichen, sich um den Abschnitt des Umfangs der Öffnung (615) zu erstrecken, wobei der äußere Umfangsabschnitt (808) des Tuchs (802) außerhalb der Öffnung (615) angeordnet ist, um einen Abschnitt einer Oberfläche der Lagerungseinrichtung (602) zu schützen.

13. Einrichtung nach Anspruch 12, wobei der Abschnitt der Oberfläche der Lagerungseinrichtung (2) einen Abschnitt eines Umfangs des Aufnahmeabschnitts der Behältereinheit umfasst.

14. Einrichtung nach einem der Ansprüche 1 bis 13, wobei der Sicherungsabschnitt (804) an dem Behälterkörper (602) gesichert ist, sodass sich das Tuch (802) um den Abschnitt des Umfangs der Öffnung (615) erstreckt, wobei der äußere Umfangsabschnitt (808) des Tuchs (802) außerhalb der Öffnung (615) angeordnet ist.

15. Verfahren zum Bereitstellen eines Organs oder Körpergewebes in einer Einrichtung zum Lagern eines Organs oder Körpergewebes nach einem vorhergehenden Anspruch, wobei das Verfahren Folgendes umfasst:
Platzieren des Organs oder Körpergewebes durch die Öffnung (615) in der Behältereinheit (6) und in den Lagerungsbereich (606) der Behältereinheit (6); und
Reißen entlang des brüchigen Abschnitts (810) des Tuchs (802), um den äußeren Umfangsabschnitt (808) des Tuchs (802) zu entfernen, nachdem das Organ oder Körpergewebe in den Lagerungsbereich (606) der Behältereinheit (6) platziert worden ist.

## Revendications

1. Appareil de stockage d'un organe ou d'un tissu corporel, l'appareil comprenant :
une unité de contenant (6) comprenant un corps de contenant (602) définissant une région de stockage (606) pour stocker un organe ou un tissu corporel, dans lequel le corps de contenant (602) inclut une ouverture (615) pour permettre à l'organe ou au tissu corporel d'être admis dans la région de stockage (606), et retiré de celle-ci, à travers l'ouverture (615) ; et
un champ chirurgical (802) comprenant : (i) une partie de fixation (804) qui est apte à être fixée à l'unité de contenant (6), (ii) une partie externe périphérique (808) qui entoure la partie de fixation (804), et (iii) une partie frangible (810) séparant la partie de fixation (804) de la partie externe périphérique (808) ;
dans lequel la partie de fixation (804) est apte à être fixée au corps de contenant (602) pour permettre au champ (802) de s'étendre autour d'une partie de la périphérie de l'ouverture (615) avec la partie externe périphérique (808) du champ (802) disposée vers l'extérieur de l'ouverture (615) ; et
dans lequel la partie frangible (810) s'étend autour de la partie de fixation (804) pour permettre à la partie externe périphérique (808) d'être séparée de la partie de fixation (804) par déchirement de la partie frangible (810).

2. Appareil selon la revendication 1, dans lequel l'unité de contenant (6) a une partie d'attache à laquelle le champ chirurgical (802) est apte à être fixé.

3. Appareil selon la revendication 2, dans lequel la partie d'attache entoure au moins une partie de l'ouverture (615) de l'unité de contenant (6).

4. Appareil selon l'une quelconque des revendications précédentes, dans lequel la partie de fixation (804) du champ (802) comprend une région adhésive pour fixer le champ (802) à l'unité de contenant (6).

5. Appareil selon l'une quelconque des revendications précédentes, dans lequel le champ (802) inclut une ouverture d'accès qui correspond à l'ouverture (615) de l'unité de contenant (6) pour permettre au champ chirurgical (802) d'être fixé à l'unité de contenant (6), l'ouverture d'accès étant alignée avec l'ouverture (615) de l'unité de contenant (6).

6. Appareil selon la revendication 5, en combinaison de la revendication 4, dans lequel la région adhésive entoure au moins une partie de l'ouverture d'accès.

7. Appareil selon l'une quelconque des revendications précédentes, dans lequel le champ chirurgical (802) comprend un premier champ chirurgical, et l'appareil comprend en outre un deuxième champ chirurgical ; et
dans lequel le deuxième champ chirurgical comprend une deuxième partie de fixation qui apte à être fixée au corps de contenant (602) pour permettre au deuxième champ de s'étendre autour d'une partie de la périphérie de l'ouverture (615).

8. Appareil selon la revendication 7, dans lequel l'unité de contenant (6) comprend une deuxième partie d'attache à laquelle le deuxième champ chirurgical est apte à être fixé.

9. Appareil selon la revendication 8, dans lequel la deuxième partie d'attache comprend une surface interne d'une paroi du corps de contenant.

10. Appareil selon l'une quelconque des revendications précédentes, dans lequel l'unité de contenant (6) comprend en outre un élément de couvercle interne qui est configuré pour être fixé au corps de contenant (602) de façon à enfermer l'organe ou le tissu corporel à l'intérieur de la région de stockage (606) entre le corps de contenant (602) et l'élément de couvercle interne, éventuellement dans lequel l'unité de contenant (6) comprend un élément de couvercle externe qui est configuré pour être fixé au corps de contenant (602) de façon à enfermer l'élément de couvercle interne entre le corps de contenant (602) et l'élément de couvercle externe.

11. Appareil selon la revendication 10, en combinaison de la revendication 7, ou selon l'une quelconque des revendications dépendantes de ladite combinaison, dans lequel l'appareil est conçu pour permettre au deuxième champ d'être fixé au corps de contenant (602) de sorte qu'une partie du deuxième champ recouvre l'élément de couvercle interne.

12. Appareil selon l'une quelconque des revendications précédentes, comprenant en outre un appareil de stockage (2) comprenant : (i) un système d'alimentation en fluide comprenant une zone de stockage de fluide de persufflation destiné à être acheminé jusqu'à un dit organe ou tissu corporel, et (ii) une partie de réception d'unité de contenant ;
dans lequel l'unité de contenant (6) est apte à être insérée de manière amovible dans la partie de réception d'unité de contenant de l'appareil de stockage (2) ; et
dans lequel la partie de fixation (804) du champ (802) est apte à être fixée au corps de contenant (602) pour permettre au champ (802) de s'étendre autour de la partie de la périphérie de l'ouverture (615), la partie externe périphérique (808) du champ (802) étant disposée vers l'extérieur de l'ouverture (615) pour protéger une partie d'une surface de l'appareil de stockage (602).

13. Appareil selon la revendication 12, dans lequel la partie de la surface de l'appareil de stockage (2) comprend une partie d'une périphérie de la partie de réception d'unité de contenant.

14. Appareil selon l'une quelconque des revendications 1 à 13, dans lequel la partie de fixation (804) est fixée au corps de contenant (602) de sorte que le champ (802) s'étend autour de la partie de la périphérie de l'ouverture (615) avec la partie externe périphérique (808) du champ (802) disposée vers l'extérieur de l'ouverture (615).

15. Procédé de fourniture d'un organe ou d'un tissu corporel dans un appareil de stockage d'un organe ou d'un tissu corporel tel que défini dans l'une quelconque des revendications précédentes, le procédé comprenant :
le positionnement de l'organe ou du tissu corporel à travers l'ouverture (615) dans l'unité de contenant (6) et dans la région de stockage (606) de l'unité de contenant (6) ; et
le déchirement du champ (802) le long de la partie frangible (810) pour retirer la partie externe périphérique (808) du champ (802) après le positionnement de l'organe ou du tissu corporel dans la région de stockage (606) de l'unité de contenant (6).
